# EUROPEAN PATENT SPECIFICATION

(11) **EP 1 413 199 B1**
(45) Date of publication and mention of the grant of the patent: **11.07.2007**
(21) Application number: 04001677.6
(22) Date of filing: 26.03.1996
(51) Int. Cl.: A01N 43/828

(54) **An agricultural and horticultural disease controller and a method for controlling the diseases**
Schädlingsbekämpfungsmittel für Landwirtschaft und Gartenbau und Verfahren zur Bekämpfung von Schädlingen
Agent de lutte contre les maladies dans les domaines de l'agriculture et de l'horticulture et procédé de lutte contre ces maladies

(30) Priority: 31.03.1995 JP 99880795
(43) Date of publication of application: 28.04.2004
(62) Divisional of application: 96906949.1
(73) Proprietor: NIHON NOHYAKU CO., LTD., Tokyo 103 (JP)
(72) Inventor: Kuroda, Kiyoshi, Ibaraki-shi, Osaka, 567-0892 (JP); Tajima, Sokichi, Osaka-shi Osaka 558 (JP); Uchikurohane, Toru, Hashimoto-shi Wakayama 648 (JP); Tsubata, Kenji, Kawachinagano-shi Osaka 586 (JP)
(74) Representative: Grünecker, Kinkeldey, Stockmair & Schwanhäusser Anwaltssozietät

(56) References cited:
- EP-A- 0 046 497
- FR-A- 2 395 263
- FR-A- 2 451 371
- FR-A- 2 453 165

## Description

### Technical Field

The present invention relates to the use of an agricultural and horticultural disease controller containing as an active ingredient a 1,2,3-thiadiazole derivative represented by the general formula (1), or a salt thereof: 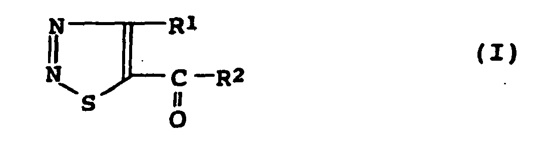 wherein R¹ is ① a hydrogen atom, ② a (C₁-C₁₂)alkyl group, ③ a halo(C₁-C₁₂)alkyl group, ④ a (C₂-C₁₂)-alkenyl group, ⑤ a halo(C₂-C₁₂)alkenyl group, ⑥ a (C₂-C₁₂)alkynyl group, ⑦ a halo(C₂-C₁₂)alkynyl group, ⑧ a (C₃-C₆)cycloalkyl group, ⑨ an unsubstituted phenyl group, ⑩ a substituted phenyl group having one or more substituents which may be the same or different and are selected from the group consisting of halogen atoms, nitro group, cyano group, (C₁-C₆)alkyl groups, halo(C₁-C₆)alkyl groups, (C₁-C₆)alkoxy groups, halo(C₁-C₆)alkoxy groups, (C₁-C₆)alkylthio groups, halo(C₁-C₆)alkylthio groups, (C₂-C₆)alkenyl groups and (C₂-C₆)alkynyl groups, ⑪ a group represented by the formula: 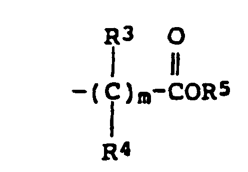 wherein R³ and R⁴, which may be the same or different, are hydrogen atoms, (C₁-C₁₂)alkyl groups or halo-(C₁-C₁₂)alkyl groups, m is zero or an integer of 1 to 6, and R⁵ is a hydrogen atom; a (C₁-C₁₂)alkyl group; a halo(C₁-C₁₂)alkyl group; a (C₂-C₁₂)alkenyl group; a (C₂-P₁₂)alkynyl group; an unsubstituted phenyl group; a substituted phenyl group having one or more substituents which may be the same or different and are selected from the group consisting of halogen atoms, nitro group, cyano group, (C₁-C₆)alkyl groups, halo(C₁-C₆)alkyl groups, (C₁-C₆)alkoxy groups, halo(C₁-C₆)alkoxy groups, (C₁-C₆)alkylthio groups, halo(C₁-C₆)alkylthio groups, (C₂-C₆)alkenyl groups and (C₂-C₆)alkynyl groups; an unsubstituted phenyl(C₁-C₆)alkyl group; or a substituted phenyl(C₁-C₆)alkyl group having on the ring one or more substituents which may be the same or different and are selected from the group consisting of halogen atoms, nitro group, cyano group, (C₁-C₆)alkyl groups, halo(C₁-C₆)alkyl groups, (C₁-C₆)alkoxy groups, halo(C₁-C₆)alkoxy groups, (C₁-C₆)alkylthio groups, halo(C₁-C₆)alkylthio groups, (C₂-C₆)alkenyl groups and (C₂-C₆)alkynyl groups, ⑫ a group represented by the formula: 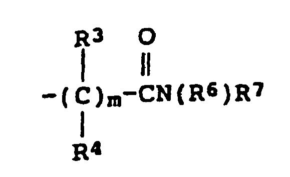 wherein R³, R⁴ and m are as defined above, and R⁶ and R⁷, which may be the same or different, are hydrogen atoms; (C₁-C₁₂)alkyl groups; halo(C₁-C₁₂)alkyl groups; (C₂-C₁₂)alkenyl groups; (C₂-C₁₂)alkynyl groups; (C₁-C₁₂)alkoxy(C₁-C₆)alkyl groups; (C₁-C₁₂)alkylthio-(C₁-C₆)alkyl groups; cyano(C₁-C₁₂)alkyl groups; unsubstituted phenyl groups; substituted phenyl groups having one or more substituents which may be the same or different and are selected from the group consisting of halogen atoms, nitro group, cyano group, (C₁-C₆)alkyl groups, halo(C₁-C₆)alkyl groups, (C₁-C₆)alkoxy groups, halo(C₁-C₆)alkoxy groups, (C₁-C₆)alkylthio groups, halo(C₁-C₆)alkylthio groups, (C₂-C₆)alkenyl groups and (C₂-C₆)alkynyl groups; unsubstituted phenyl(C₁-C₆)alkyl groups; or substituted phenyl(C₁-C₆)alkyl groups having on the ring one or more substituents which may be the same or different and are selected from the group consisting of halogen atoms, nitro group, cyano group, (C₁-C₆)alkyl groups, halo(C₁-C₆)alkyl groups, (C₁-C₆)alkoxy groups, halo(C₁-C₆)alkoxy groups, (C₁-C₆)alkylthio groups, halo(C₁-C₆)alkylthio groups, (C₂-C₆)alkenyl groups and (C₂-C₆)alkynyl groups; R⁶ and R⁷ being able to be taken together to represent a (C₄-C₆)alkylene group which may contain an oxygen atom between adjacent carbon atoms of the carbon chain, ⑬ a group represented by the formula: 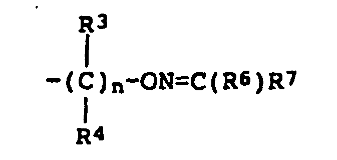 wherein R³, R⁴, R⁶ and R⁷, and n is an integer of 1 to 6, or ⑭ a group represented by the formula: 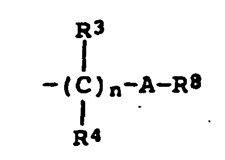 wherein R³, R⁴ and n are as defined above, A is
-O- , 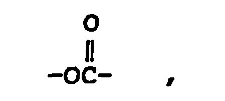 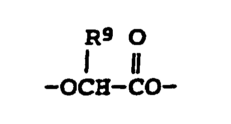 wherein R⁹ is a hydrogen atom, a (C₁-C₁₂)alkyl group or a halo(C₁-C₁₂)alkyl group,
-S-,
-SO-,
-SO₂-, 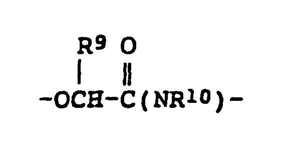 wherein R⁹ is as defined above, and R¹⁰ is a hydrogen atom, a (C₁-C₁₂)alkyl group or a halo(C₁-C₁₂)alkyl group, R⁹ and R¹⁰ being able to be taken together to represent a (C₄-C₆)alkylene group which may contain, between adjacent carbon atoms of the carbon chain, an oxygen atom, a sulfur atom or
N-R¹¹
(wherein R¹¹ is a hydrogen atom, a (C₁-C₁₂)alkyl group or a halo(C₁-C₁₂)alkyl group, or
-N(R¹⁰)
wherein R¹⁰ is as defined above, and R⁸ is a hydrogen atom; a (C₁-C₁₂)alkyl group; a halo(C₁-C₁₂)alkyl group; a (C₂-C₁₂)alkenyl group; a (C₂-C₁₂)alkynyl group; a (C₁-C₁₂)alkoxy(C₁-C₁₂)alkyl group; a (C₁-C₁₂)alkylthio-(C₁-C₁₂)alkyl group; a cyano(C₁-C₁₂)alkyl group; an unsubstituted phenyl group; a substituted phenyl group having one or more substituents which may be the same or different and are selected from the group consisting of halogen atoms, nitro group, cyano group, (C₁-C₆)alkyl groups, halo(C₁-C₆)alkyl groups, (C₁-C₆)alkoxy groups, halo(C₁-C₆)alkoxy groups, (C₁-C₆)alkylthio groups, halo(C₁-C₆)alkylthio groups, (C₂-C₆)alkenyl groups and (C₂-C₆)alkynyl groups; an unsubstituted phenyl(C₁-C₆)-alkyl group; a substituted phenyl(C₁-C₆)alkyl group having on the ring one or more substituents which may be the same or different and are selected from the group consisting of halogen atoms, nitro group, cyano group, (C₁-C₆)alkyl groups, halo(C₁-C₆)alkyl groups, (C₁-C₆)-alkoxy groups, halo(C₁-C₆)alkoxy groups, (C₁-C₆)alkyl-thio groups, halo(C₁-C₆)alkylthio groups, (C₂-C₆)alkenyl groups and (C₂-C₆)alkynyl groups; an unsubstituted 5- or 6-membered heterocyclic ring having one or more heteroatoms which may be the same or different and are selected from the group consisting of oxygen atom, sulfur atom and nitrogen atom; or a substituted 5- or 6-membered heterocyclic ring having one or more heteroatoms which may be the same or different and are selected from the group consisting of oxygen atom, sulfur atom and nitrogen atom, said substituted heterocyclic ring having one or more substituents which may be the same or different and are selected from the group consisting of halogen atoms, nitro group, cyano group, (C₁-C₆)alkyl groups, halo(C₁-C₆)alkyl groups, (C₁-C₆)-alkoxy groups, halo(C₁-C₆)alkoxy groups, (C₁-C₆)alkyl-thio groups, halo(C₁-C₆)alkylthio groups, (C₂-C₆)alkenyl groups and (C₂-C₆)alkynyl groups, and R² is
a group represented by the formula:

-N(R²¹)R²²

wherein R²¹ is a hydrogen atom and R²² is selected from ② (C₁-C₁₂)alkyl groups, ③ unsubstituted halo(C₁-C₁₂)alkyl groups, ④ substituted halo(C₁-C₁₂)alkyl groups having a hydroxyl group or a (C₁-C₆)alkoxy group as the substituent, ⑤ (C₂-C₁₂)alkenyl groups, ⑥ (C₂-C₁₂)alkynyl groups, ⑦ (C₁-C₁₂)alkoxy(C₁-C₆)alkyl groups, ⑧ (C₁-C₁₂)alkylthio-(C₁-C₆)alkyl groups, ⑨ cyano(C₁-C₁₂)alkyl groups, ⑩ substituted cyano(C₁-C₆)alkyl groups having on the alkyl chain a substituent selected from the group consisting of (C₁-C₆)alkoxy groups, (C₂-C₆)alkenyloxy groups, (C₂-C₆)alkynyloxy groups, (C₁-C₆)alkylthio groups, (C₁-C₆)alkoxycarbonyl(C₁-C₆)alkoxy groups, (C₁-C₆)-alkoxycarbonyl(C₁-C₆)alkylthio groups, phenoxy group, phenylthio group and pyrazol-1-yl group, ⑬ hydroxy-(C₁-C₆)alkyl groups, ⑭di(C₁-C₆)alkoxy(C₁-C₆)alkyl groups in which the (C₁-C₆)alkoxy groups may be the same or different, ⑮ unsubstituted (C₁-C₆)alkoxycarbonyl-(C₁-C₆)alkyl groups, ⑯ substituted (C₁-C₆)alkoxycarbonyl(C₁-C₆)alkyl groups having on the alkyl chain a substituent selected from the group consisting of (C₁-C₆)alkyl groups, (C₁-C₆)alkoxy groups, (C₂-C₆)-alkenyloxy groups, (C₂-C₆)alkynyloxy groups, (C₁-C₆)-alkylthio groups, (C₁-C₆)alkoxycarbonyl(C₁-C₆)alkoxy groups, (C₁-C₆)alkoxycarbonyl(C₁-C₆)alkylthio groups, phenyl group, phenyl(C₁-C₆)alkyl group, phenoxy group, phenylthio group and pyrazol-1-yl group, ⑰ unsubstituted phenyl groups, ⑱ substituted phenyl groups having one or more substituents which may be the same or different and are selected from the group consisting of ¹⁾ halogen atoms, ²⁾ nitro group, ³⁾ cyano group, ⁴⁾ (C₁-C₆)alkyl groups, ⁵⁾ halo(C₁-C₆)alkyl groups, ⁶⁾ (C₁-C₆)alkoxy groups, ⁷⁾ halo(C₁-C₆)alkoxy groups, ⁸⁾ (C₁-C₆)alkylthio groups, ⁹⁾ halo(C₁-C₆)alkylthio groups, ¹⁰⁾ (C₂-C₆)alkenyl groups, ¹¹⁾ (C₂-C₆)alkynyl groups, ¹²⁾ (C₁-C₆)alkylcarbonyl groups, ¹³⁾ carboxyl group, ¹⁴⁾ (C₁-C₁₂)alkoxycarbonyl groups, ¹⁵) methylenedioxy group, ¹⁶⁾ phenyl group, ¹⁷) groups represented by the formula: 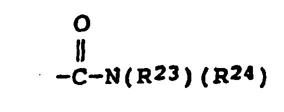 wherein R²³ and R²⁴, which may be the same or different, are hydrogen atoms; (C₁-C₁₂)alkyl groups; halo-(C₁-C₁₂)alkyl groups; (C₂-C₁₂)alkenyl groups; (C₂-C₁₂)-alkynyl groups; unsubstituted phenyl groups; substituted phenyl groups having one or more substituents which may be the same or different and are selected from the group consisting of halogen atoms, cyano group, nitro group, (C₁-C₆)alkyl groups and (C₁-C₆)alkoxy groups; or 5- or 6-membered heterocyclic rings containing, between adjacent carbon atoms of the carbon chain, one or more hetero atoms which may be the same or different and are selected from the group consisting of oxygen atom, sulfur atom and nitrogen atom, ¹⁸⁾ groups represented by the formula:

-SO₂-R²⁵

wherein R²⁵ is a hydrogen atom, hydroxyl group, a (C₁-C₁₂)alkyl group, a halo(C₁-C₆)alkyl group, an unsubstituted phenyl group, a substituted phenyl group having as the substituent(s) one or more substituents which may be the same or different and are selected from the group consisting of halogen atoms, (C₁-C₆)alkyl groups and (C₁-C₆)alkoxy groups, unsubstituted 1,2,3-thiadiazol-5-yl group, or substituted 1,2,3-thiadiazol-5-yl groups having halogen atom, (C₁-C₆)alkyl group or (C₁-C₆)alkoxy group as the substituent, ¹⁹⁾ groups represented by the formula: 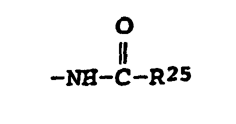 wherein R²⁵ is as defined above, ²⁰⁾ groups represented by the formula: 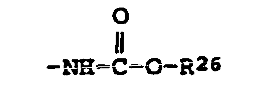 (wherein R²⁶ is a (C₁-C₆)alkyl group, a phenyl group or a phenyl(C₁-C₆)alkyl group , ²¹⁾ groups represented by the formula:

-NH-SO₂-R²⁵

wherein R²⁵ is as defined above: , ²²⁾ groups represented by the formula: 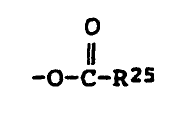 wherein R²⁵ is as defined above , and ²³⁾ groups represented by the formula: 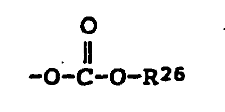 wherein R²⁶ is as defined above, ⑲ unsubstituted phenyl(d₁-C₆)alkyl groups, ⑳ substituted phenyl(C₁-C₆)-alkyl groups having on the ring one or more substituents which may be the same or different and are selected from the group consisting of halogen atoms, nitro group, cyano group, (C₁-C₆)alkyl groups, halo(C₁-C₆)alkyl groups, (C₁-C₆)alkoxy groups, halo(C₁-C₆)alkoxy groups, (C₁-C₆)alkylthio groups, halo(C₁-C₆)alkylthio groups, (C₂-C₆)alkenyl groups and (C₂-C₆)alkynyl groups, 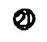 naphthyl groups, 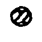 groups represented by the formula:

-(CH₂)ₚNHR18

wherein R¹⁸ is as defined above, and p is an integer of 1 to 12 , or 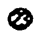 5- or 6-membered heterocyclic rings having one or more hetero atoms which may be the same or different and are selected from the group consisting of oxygen atom, sulfur atom and nitrogen atom, R²¹ and R²² being able to be taken together to represent 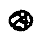 a (C₄-C₆)alkylene group which may contain an oxygen atom between adjacent carbon atoms of the carbon chain, or 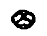 a diaminomethylene group
for controlling agricultural and horticultural diseases which damage crops, fruit trees, vegetables, flowers and ornamental plants.

### Background Art

Japanese Patent Unexamined Publication No. 54-9272 discloses 1,2,3-thiadiazole-5-carboxylic acid derivatives, a process for production thereof and an agent containing the derivative and having herbicidal and growth-regulating effects.

FR-A-2 453 165 discloses 1,2,3-thiadiazole-5-carboxamides and their use as herbicidal products, growth regulators, defoliants and fungicides.

EP-A-0 046 497 relates to N-disubstituted aniline derivatives, their production and their use as microbicides.

FR-A-2 451 371 discloses 1,2,3-thiadiazole-5-carboxylic acid derivatives, a process for the production thereof and an agent containing the derivative and having herbicidal and fungicidal effects.

### Disclosure of Invention

The present inventors earnestly investigated for developing a novel agricultural and horticultural disease controller and consequently found that 1,2,3-thiadiazole derivatives or salts thereof, which include some of the compounds disclosed in USP 4177054, are useful for herbicide and growth regulator, whereby the present invention has been accomplished.

### Best Mode for Carrying Out the Invention

In the definition of the substituents of the 1,2,3-thiadiazole derivative of the general formula (I) used as the active ingredient of the agricultural and horticultural disease controller of the present invention, the halogen atom includes chlorine atom, bromine atom, iodine atom and fluorine atom. The term "(C₁-C₁₂)alkyl group" means a linear or branched alkyl group of 1 to 12 carbon atoms, such as methyl, ethyl, n-propyl, i-propyl, n-butyl, i-butyl, s-butyl, t-butyl, n-pentyl, n-hexyl, n-heptyl, n-octyl, n-nonyl, n-decyl, n-undecyl, or n-dodecyl. The term "halo-(C₁-C₁₂)alkyl group" means a substituted and linear or branched alkyl group of 1 to 12 carbon atoms having as the substituent(s) one or more halogen atoms which may be the same or different. The term "(C₂-C₆)alkenyl group" means a linear or branched alkenyl group of 2 to 6 carbon atoms having a double bond. The term "halo-(C₂-C₆)alkenyl group" means a substituted and linear or branched alkenyl group of 2 to 6 carbon atoms having as the substituent(s) one or more halogen atoms which may be the same or different. The term "(C₂-C₆)alkynyl group" means a linear or branched alkynyl group of 2 to 6 carbon atoms having a triple bond. The term "halo-(C₂-C₆)alkynyl group" means a substituted and linear or branched alkynyl group of 2 to 6 carbon atoms having as the substituent(s) one or more halogen atoms which may be the same or different.

The term "5- or 6-membered heterocyclic ring having one or more heteroatoms which may be the same or different and are selected from the group consisting of oxygen atom, sulfur atom and nitrogen atom" means any heterocyclic ring derived from furan, thiophene, pyrrole, oxazole, thiazole, isothiazole, pyrazole, imidazole, 1,2,3-thiadiazole, 1,2,4-thiadiazole, 1,2,5-thiadiazole, 1,3,4-thiadiazole, 1,2,4-triazole, pyridine, pyridazine, pyrimidine, pyrazine, pyrrolidine, piperidine, morpholine, thiamorpholine, dithiolane, di-thian, piperazine, dioxelan, or imidazolizine.

Preferable examples of the substituents of the 1,2,3-thiadiazole derivative of the general formula (I) used in the present invention are as follows. There is preferably used a compound in which R¹ is ① a hydrogen atom, ② a (C₁-C₁₂)-alkyl group, ③ a halo(C₁-C₁₂)alkyl group, ④ a (C₂-C₁₂)alkenyl group, ⑤ a halo(C₂-C₁₂)alkenyl group, ⑥ a (C₂-C₁₂)alkynyl group, ⑦ a halo(C₂-C₁₂)alkynyl group, ⑧ a (C₃-C₆)cycloalkyl group, ⑨ an unsubstituted phenyl group or ⑩ a substituted phenyl group having one or more substituents which may be the same or different and are selected from the group consisting of halogen atoms, nitro group, cyano group (C₁-C₆)alkyl groups, halo(C₁-C₆)alkyl groups, (C₁-C₆)alkoxy groups, halo(C₁-C₆)alkoxy groups, (C₁-C₆)alkylthio groups, halo(C₁-C₆)alkylthio groups, (C₂-C₆)alkenyl groups and (C₂-C₆)alkynyl groups, and R² is a group represented by the formula:

-N(R²¹)R²²

wherein R²¹ is a hydrogen atom and R²² is selected from ② (C₁-C₁₂)alkyl groups, ③ unsubstituted halo(C₁-C₁₂)alkyl groups, ⑤ (C₂-C₁₂)alkenyl groups, ⑥ (C₂-C₁₂)alkynyl groups, ⑦ (C₁-C₁₂)alkoxy(C₁-C₆)alkyl groups, ⑧ (C₁-C₁₂)alkylthio-(C₁-C₆)alkyl groups, ⑨ cyano(C₁-C₁₂)alkyl groups, ⑬ hydroxy-(C₁-C₆)alkyl groups, ⑮ unsubstituted (C₁-C₆)alkoxycarbonyl(C₁-C₆)alkyl groups, ⑰ unsubstituted phenyl groups, ⑱ substituted phenyl groups having one or more substituents which may be the same or different and are selected from the group consisting of ¹⁾ halogen atoms, ²⁾ nitro group, ³⁾ cyano group, ⁴⁾ (C₁-C₆)alkyl groups, ⁵⁾ halo(C₁-C₆)alkyl groups, ⁶⁾ (C₁-C₆)alkoxy groups, ⁷⁾ halo (C₁-C₆) alkoxy groups, ⁸⁾ (C₁-C₆)alkylthio groups, ¹²⁾ (C₁-C₆)alkylcarbonyl groups, ¹³⁾ carboxyl group, ¹⁴⁾ (C₁-C₁₂)alkoxycarbonyl groups, ¹⁵⁾ methylenedioxy group, ¹⁶⁾ phenyl group, ¹⁷⁾ groups represented by the formula: 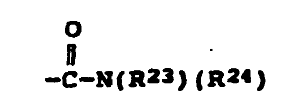 wherein R²³ and R²⁴, which may be the same or different, are hydrogen atoms; (C₁-C₁₂)alkyl groups; halo-(C₁-C₁₂)alkyl groups ; (C₂-C₁₂)alkenyl groups; (C₂-C₁₂)-alkynyl groups; unsubstituted phenyl groups; substituted phenyl groups having one or more substituents which may be the same or different and are selected from the group consisting of halogen atoms, cyano group, nitro group, (C₁-C₆)alkyl groups and (C₁-C₆)alkoxy groups; or 5- or 6-membered heterocyclic rings containing, between adjacent carbon atoms of the carbon chain, one or more atoms which may be the same or different and are selected from the group consisting of oxygen atom, sulfur atom and nitrogen atom, ¹⁸⁾ groups represented by the formula:

-SO₂-R²⁵

wherein R²⁵ is a hydrogen atom, hydroxyl group, a (C₁-C₆)alkyl group, a halo(C₁-C₆)alkyl group, an unsubstituted phenyl group, a substituted phenyl group having as the substituent(s) one or more substituents which may be the same or different and are selected from the group consisting of halogen atoms, (C₁-C₆)alkyl groups and (C₁-C₆)alkoxy groups, unsubstituted 1,2,3-thiadiazol-5-yl group, or substituted 1,2,3-thiadiazol-5-yl groups having halogen atom, (C₁-C₆)alkyl group or (C₁-C₆)alkoxy group as the substituent, ¹⁹⁾ groups represented by the formula: 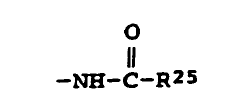 wherein R²⁵ is as defined above, ²⁰⁾ groups represented by the formula: 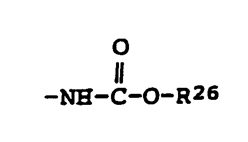 wherein R²⁶ is a (C₁-C₆)alkyl group, a phenyl group or a phenyl(C₁-C₆)alkyl group), ²¹⁾ groups represented by the formula:

-NH-SO₂-R²⁵

wherein R²⁵ is as defined above , ²²⁾ groups

represented by the formula: 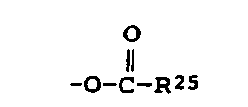 wherein R²⁵ is as defined above, and ²³⁾ groups represented by the formula: 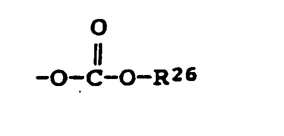 wherein R²⁶ is as defined above, ⑲ unsubstituted phenyl(C₁-C₆)alkyl groups, ⑳ substituted phenyl(C₁-C₆)-alkyl groups having on the ring one or more substituents which may be the same or different and are selected from the group consisting of halogen atoms, nitro group, cyano group, (C₁-C₆)alkyl groups, halo(C₁-C₆)alkyl groups, (C₁-C₆)alkoxy groups, and halo(C₁-C₆)alkoxy groups, 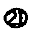 naphthyl groups, 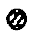 groups represented by the formula:

-(CH₂)ₚNHR¹⁸

(wherein R¹⁸ is as defined above, and p is an integer of 1 to 12, or 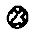 5- or 6-membered heterocyclic rings having one or more atoms which may be the same or different and are selected from the group consisting of oxygen atom, sulfur atom and nitrogen atom, R²¹ and R²² being able to be taken together to represent 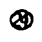 a (C₄-C₆)alkylene group which may contain an oxygen atom between adjacent carbon atoms of the carbon chain, or 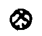 a diaminomethylene group.

As the salt of the 1,2,3-thiadiazole derivative of the general formula (I), there can be exemplified salts with alkali metals such as sodium, potassium, etc.; salts with alkaline earth metals such as calcium, magnesium, etc.; unsubstituted ammonium salt; substituted ammonium salts having one or more substituents which may be the same or different and are selected from the group consisting of (C₁-C₁₂)alkyl groups, unsubstituted phenyl group, substituted phenyl groups, unsubstituted benzyl group and substituted benzyl groups; and guanidium salt.

The 1,2,3-thiadiazole derivative of the general formula (I) or salt thereof used as the active ingredient of the agricultural and horticultural disease controller of the present invention can be produced, for example, by any of the processes exemplified below: 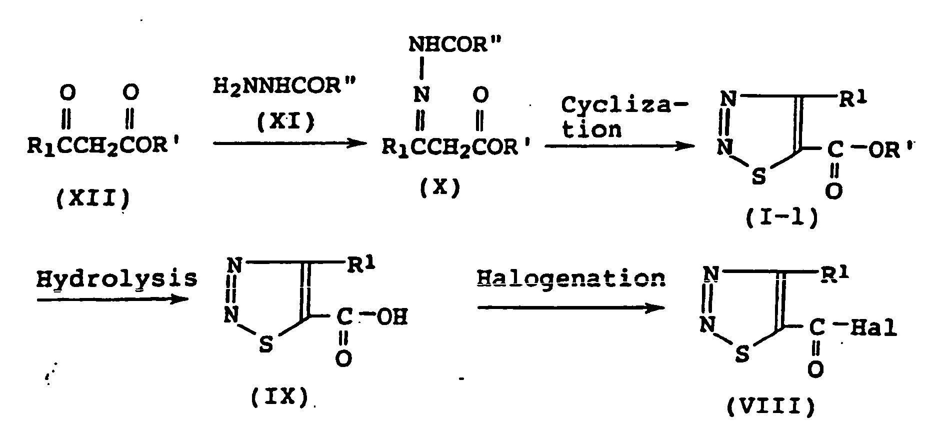 wherein R¹ is as defined above, R' is a (C₁-C₆)alkyl group, R" is an amino group or a (C₁-C₆)alkyl group, and Hal is a halogen atom.

A compound of the general formula (XII) is reacted with a compound of the general formula (XI) to obtain a compound of the general formula (X). The compound (X) is cyclized after or without isolation to obtain a 1,2,3-thiadiazole derivative of the general formula (1-1). The derivative (I-1) is hydrolyzed after or without isolation to obtain a compound of the general formula (IX). The compound (IX) is halogenated after or without isolation, whereby the acid halide of the general formula (VIII) can be produced.

The compound of the general formula (XII) can be produced by the process described in J. Org. Chem., 43, 2087 (1978), Formation of C-C bonds, Vol. 3, p. 259, 1979 (Georg Thime Publishers, Stuttgart), etc.

From the acid halide of the general formula (VIII) produced by the above process, the 1,2,3-thiadiazole derivative of the general formula (I) or salt thereof used as the active ingredient of the agricultural and horticultural disease controller of the present invention can be produced, for example, by any of the processes illustrated below. 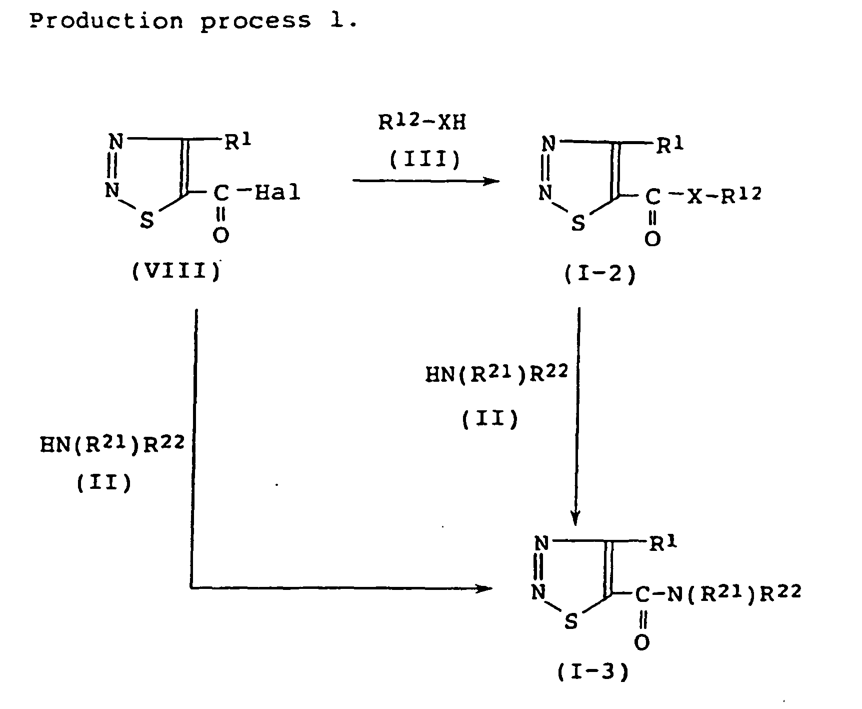 wherein R¹, R¹², R²¹, R²² and Hal are as defined above.

The acid halide of the general formula (VIII) is reacted with an alcohol of the general formula (III) to obtain a 1,2,3-thiadiazole derivative of the general formula (I-2). The compound (I-2) is reacted with an amine of the general formula (II) after or without isolation of the compound (I-2), whereby a 1,2,3-thiadiazole derivative of the general formula (I-3) can be produced.

The 1,2,3-thiadiazole derivative of the general formula (I-3) can be produced also by reacting the acid halide of the general formula (VIII) with an amine of the general formula (II). 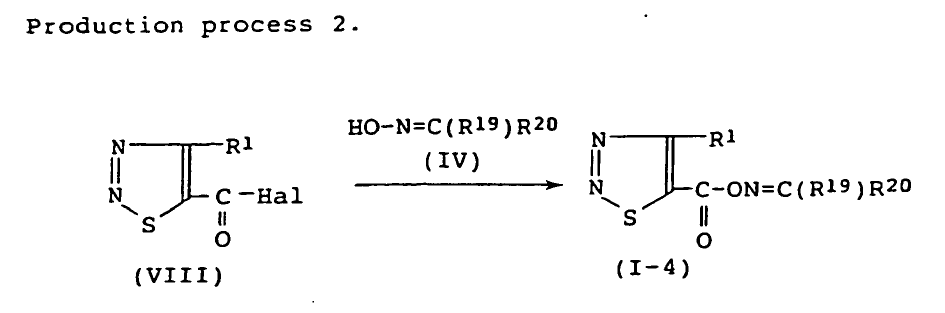 wherein R¹⁹, R²⁰ and Hal are as defined above.

A 1,2,3-thiadiazole derivative of the general formula (1-4) can be produced by reacting the acid halide of the general formula (VIII) with a compound of the general formula (IV). 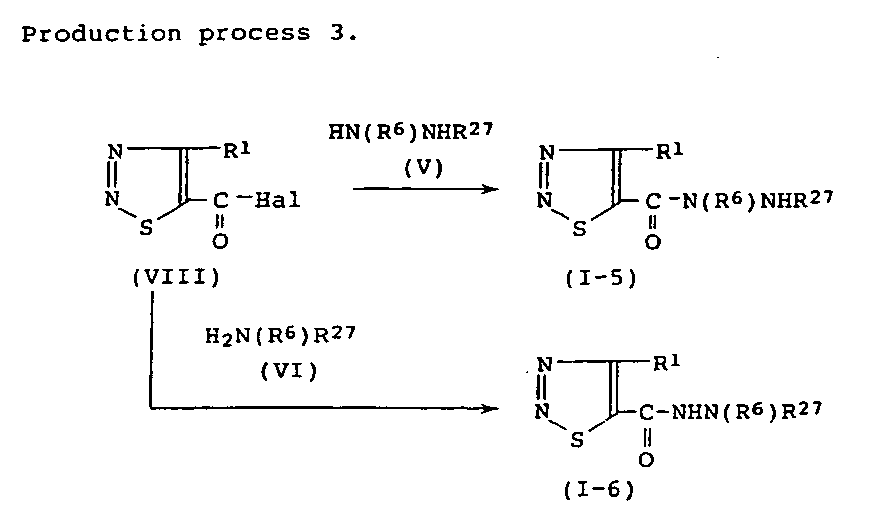 wherein R⁶, R²⁷ and Hal are as defined above.

A 1,2,3-thiadiazole derivative of the general formula (I-5) can be produced by reacting the acid halide of the general formula (VIII) with a compound of the general formula (V). Similarly, a 1,2,3-thiadiazole derivative of the general formula (I-6) can be produced by reacting the acid halide of the general formula (VIII) with a compound of the general formula (VI). 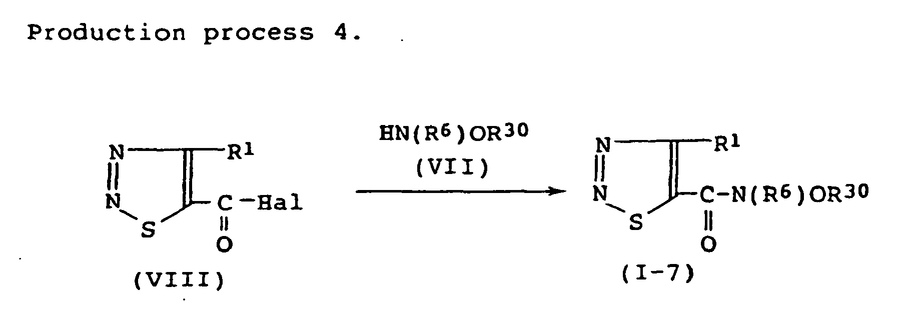 wherein R⁶, R³⁰ and Hal are as defined above.

A 1,2,3-thiadiazole derivative of the general formula (I-7) can be produced by reacting the acid halide of the general formula (VIII) with a compound of the general formula (VII).

Typical compounds as the 1,2,3-thiadiazole derivative of the general formula (I) or a salt thereof are listed in Table 1 but they are not intended in any way to limit the scope of the present invention.

The abbreviations in the R¹ column and R² column in Table 1 stand for the following compounds:

| | | | | | |
|---|---|---|---|---|---|
| Ph: | 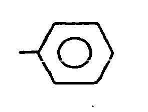 | naph: | 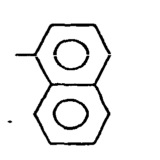 | Q₁: | 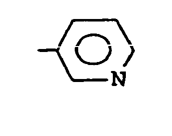 |
| Q₂ : | 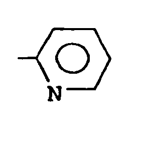 | Q₃: | 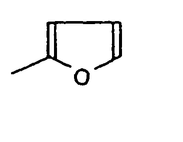 | Q₄: | 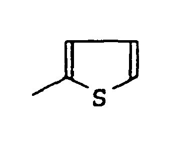 |
| Q₅: | 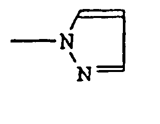 | Q₆: | 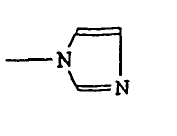 | Q₇ : | 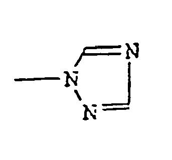 |
| Q₈: | 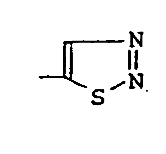 | Q₉: | 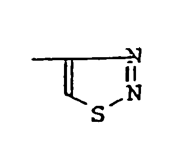 | Q₁₀: | 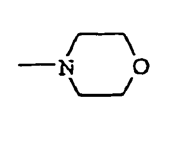 |
| Q₁₁: | 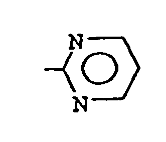 | Q₁₂: | 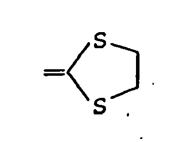 | Q₁₃: | 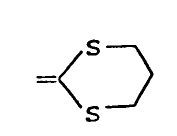 |
| Q₁₄: | 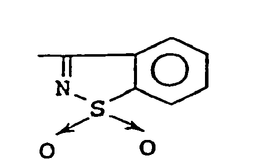 | Q₁₅: | | 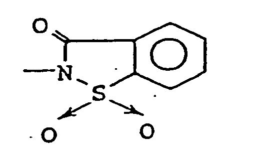 | |
| Q₁₆: | 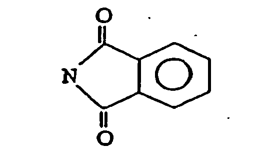 | Q₁₇: | | 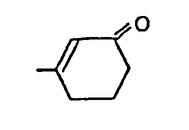 | |
| Q₁₈: | 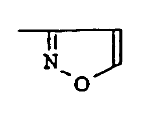 | Q₁₉: | 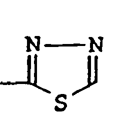 | Q₂₀: | 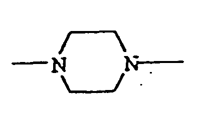 |
| Q₂₁: | 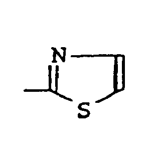 | Q₂₂: | 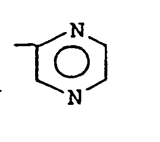 | | |

General formula (I):

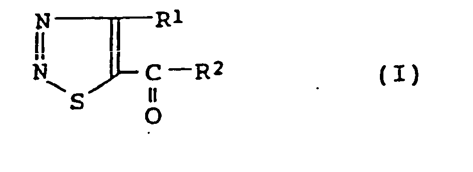

**Table 1**

| No | R¹ | R² | Physical property |
|---|---|---|---|
| 4 | H | NH-Ph | m.p. 132.0°C |
| 5 | H | NH-CH(Q₅)-CN | m.p. 163.9°C |
| 6 | H CH₃ | NHOCH₃ | m.p. 113.1°C |
| 85 | CH₃ | NHCH₃ | m.p. 45°C |
| 86 | CH₃ | N(CH₃)₂ | nD 1.5555 (13.2°C) |
| 87 | CH₃ | NHC₂H₅ | m.p. 44°C |
| 88 | CH₃ | N(C₂H₅)₂ | nD 1.5365 (13.7°C) |
| 89 | CH₃ | NHC₃H₇-i | m.p. 65°C |
| 90 | CH₃ | NRC₄H₉-n | |
| 91 | CH₃ | NHC₆H₁₁-cyclo | m.p. 98°C |
| 92 | CH₃ | NHCH₂CH₂OCH₃ | nD 1.5359 (26.0°C) |
| 93 | CH₃ | NHCH₂CH₂CH₂OCH₃ | nD 1.5273 (26.0°C) |
| 94 | CH₃ | NHCH₂CH(OCH₃)₂ | nD 1.5240 (26.1°C) |
| 95 | CH₃ | N(CH₂CH=CH₂)₂ | nD 1.5535 (27.5°C) |
| 96 | CH₃ | NHC(CH₃)₂-C≡CH | NMR |
| 97 | CH₃ | NHCH(CH₃)CH₂OH | NMR |
| 98 | CH₃ | NHCH₂CN | m.p. 76-78°C |
| 99 | CH₃ | NHCH₂CH₂CN | m.p. 86-87°C |
| 100 | CH₃ | N(CH₂CH₂CN)₂ | m.p. 110-115°C |
| 101 | CH₃ | NHC(CH₃)(i-C₃H₇)-CN | nD 1.5235 (25.9°C) |
| 102 | CH₃ | NHCH(C₃H₇-i)COOCH₃ | NMR |
| 103 | CH₃ | NHCH(CH₃)CH₂COOC₂H₅ | nD 1.5250 (20.4°C) |
| 104 | CH₃ | NHPh | m.p. 110°C |
| 105 | CH₃ | NH(2-Cl-Ph) | m.p. 101°C |
| 106 | CH₃ | NH(3-Cl-Ph) | m.p. 136-142°C |
| 107 | CH₃ | NH(4-Cl-Ph) | m.p. 114°C |
| 108 | CH₃ | NH(2-F-Ph) | m.p. 120°C |
| 109 | CH₃ | NH(3-F-Ph) | m.p. 127°C |
| 110 | CH₃ | NH(4-F-Ph) | m.p. 103°C |
| 111 | CH₃ | NH(2-CH₃-Ph) | m.p. 115°C |
| 112 | CH₃ | NH(3-CH₃-Ph) | m.p. 111°C |
| 113 | CH₃ | NH(4-CH₃-Ph) | m.p. 109°C |
| 114 | CH₃ | NH(3-i-C₃H₇-Ph) | NMR |
| 115 | CH₃ | NH(2-OCH₃-Ph) | m.p. 112°C |
| 116 | CH₃ | NH(3-OCH₃-Ph) | m.p. 107-110°C |
| 117 | CH₃ | NH(4-OCH₃-Ph) | m.p. 117°C |
| 118 | CH₃ | NH(3-O-i-C₃H₇-Ph) | NMR |
| 119 | CH₃ | NH(4-NO₂-Ph) | m.p. 175°C |
| 120 | CH₃ | NH(3-CN-Ph) | m.p. 161°C |
| 121 | CH₃ | NH(4-CN-Ph) | m.p. 172°C |
| 122 | CH₃ | NH(4-CO₂H-Ph) | m.p. 257°C (decomp.) |
| 123 | CH₃ | NH(4-CO₂CH₃-Ph) | m.p. 133°C |
| 124 | CH₃ | NH(4-CO₂C₂H₅-Ph) | m.p. 118°C |
| 125 | CH₃ | NH(3-COCH₃-Ph) | m.p. 154°C |
| 126 | CH₃ | NH(4-COCH₃-Ph) | m.p. 128°C |
| 127 | CH₃ | NH(2-Ph-Ph) | m.p. 85.5°C |
| 128 | CH₃ | NH(2,4-Cl₂-Ph) | m.p. 118-119°C |
| 129 | CH₃ | NH(2,5-Cl₂-Ph) | m.p. 151-155°C |
| 130 | CH₃ | NH(3,4-Cl₂-Ph) | m.p. 138-139°C |
| 131 | CH₃ | NH(3,5-Cl₂-Ph) | m.p. 197-199°C |
| 132 | CH₃ | NH(2,4-(CH₃)₂-Ph) | m.p. 98.3-98.9°C |
| 133 | CH₃ | NH(2,6-(CH₃)₂-Ph) | m.p. 95-99°C |
| 134 | CH₃ | NH(3,5-(CF₃)₂-Ph) | m.p. 170°C |
| 135 | CH₃ | NH(3-OCH₂O-4-Ph) | m.p. 138°C |
| 136 | CH₃ | NH(2,4,6-(CH₃)₃-Ph) | m.p. 115-117°C |
| 137 | CH₃ | NH(2,6-Br₂-4-OCF₃-Ph) | m.p. 151.7°C |
| 138 | CH₃ | NH(F₅-Ph) | m.p. 124°C |
| 139 | CH₃ | NH-CH₂-Ph | m.p. 53°C |
| 140 | CH₃ | N(CH₃)-Ph | paste NMR |
| 141 | CH₃ | NHC(CH₃)₂-Ph | m.p. 139°C |
| 142 | CH₃ | NHCH₂-(4-Cl-Ph) | m.p. 102-105°C |
| 143 | CH₃ | NHCH(CH₃)(4-Cl-Ph) | m.p. 108°C |
| 144 | CH₃ | NHCH(CH₂-Ph)COOCH₃ | NMR |
| 145 | CH₃ | NH-naph | m.p. 151°C |
| 146 | CH₃ | NH-Q₂ | m.p. 130°C |
| 147 | CH₃ | Q₁₀ | m.p. 92°C |
| 148 | CH₃ | 2,6-(CH₃)₂-Q₁₀ | m.p. 96-100°C |
| | | (cis) | |
| 149 | CH₃ | NHCH₂-Q₃ | nD 1.5731 (26.1°C) |
| 150 | CH₃ | NRCH(OC₂H₅)-CN | paste NMR |
| 151 | CH₃ | NHCH(OCH₂C≡CH)-CN | NMR |
| 152 | CH₃ | NHCH(Q₅)-CN | m.p. 157.3°C |
| 153 | CH₃ | NHCH(3,5-(CH₃)₂-Q₅)-CN | m.p. 155.6°C |
| 154 | CH₃ | NRCH(SCH(CH₃)-C₂H₅)-CN | paste NMR |
| 155 | CH₃ | NHCH(S-Ph)-CN | m.p. 116.5°C |
| 156 | CH₃ | NHSO₂-Ph | m.p. 170-176°C |
| 162 | CH₃ | NH-Q₁₀ | m.p. 191. 0°C |
| 173 | CH₃ | NHOCH₃ | m.p. 70°C |
| 174 | CH₃ | NHOCH₂-Ph | m.p. 73°C |
| 183 | C₂H₅ | NH-Ph | m.p. 81.9°C |
| 192 | i-C₃H₇ | NH-Ph | m.p. 112.3°C |
| 193 | i-C₃H₇ | NH-CH₂-CN | paste NMR |
| 194 | i-C₃H₇ | NH-CH(Q₅)-CN | m.p. 107.4°C |
| 211 | CF₃ | NH-Ph | m.p. 142.7°C |
| 212 | CF₃ | NH(3-i-C₃H₇-Ph) | m.p. 81.3°C |
| 213 | CF₃ | NH(3-i-C₃H₇O-Ph) | paste NMR |
| 214 | CF₃ | NH-(3,5-(CF₃)₂-Ph) | m.p. 156.2°C |
| 215 | CF₃ | NH-CH(Q₅)-CN | m.p. 118.9°C |
| 216 | CF₃ | NHOCH₃ | m.p. 101.9°C |
| 226 | cyclo-C₃H₅ | NH-PH | m.p. 138.8°C |
| 227 | cyclo-C₃H₅ | NH-(3-i-C₃H₇-Ph) | paste NMR |
| 228 | cyclo-C₃H₅ | NH(3-i-C₃H₇0-Ph) | paste NMR |
| 236 | COOH | NHC(CH₃)(i-C₃H₇)-CN | nD 1.4832 (25.1°C) |
| 240 | COOC₂H₅ | NH-Ph | m.p. 125.4°C |
| 242 | COOC₄H₉-n | NHCH₂-Ph | paste NMR |
| 243 | CONHCH₃ | NHCH₃ | m.p. 195-196°C |
| 244 | CONHC₃H₇-i | NRC₃H₇-I | m.p. 92-96°C |
| 245 | CONHCH₂CH₂OCH₃ | NHCH₂CH₂OCH₃ | m.p. 90-92°C |
| 247 | CONHCH₂Ph | NHCH₂Ph | m.p. 113.8-116.3°C |
| 252 | CH₂CONHC₃H₇-i | NHC₃H₇-i | m.p. 146-156°C |
| 253 | CH₂CONHC₅H₁₁-i | NHC₅H₁₁-i | m.p. 84-86°C |
| 270 | Ph | NH-CH₂-CN | m.p. 147.1°C |
| 271 | Ph | NH-CH(Q₅)-CN | m.p. 146.3°C |
| 285 | CH₂SO₂Ph | NHCH(CH₃)-Ph | m.p. 127-131°C |
| 288 | CH₃ | NHCH(OC₂H₅)COOC₂H₅ | m.p. 68.8°C |
| 289 | CH₃ | NHCH(OC₃H₇-i)COOC₂H₅ | m.p. 52.1°C |
| 290 | CH₃ | NKCH(Q₅)COOC₂H₅ | m.p. 107.3°C |
| 291 | CH₃ | NHCH(OC₂R₅)CONH₂ | m.p. 136.9°C |
| 292 | CH₃ | NRCH(CN)OCH₂COOC₂H₅ | nD 1.5217 (21.7°C) |
| 293 | CH₃ | NHCH(CN)SCH₂COOC₂H₅ | nD 1.5525 (21.5°C) |
| 294 | CH₃ | NHCH₂COOC₂H₅ | nD 1.5248 (21.5°C) |
| 295 | CH₃ | NHCH(CCl₃)OH | m.p. 150°C |
| 296 | CH₃ | NHCH(CCl₃)OC₄H₉-n | paste NMR |
| 340 | CH₃ | NH(3-C₂H₅-Ph) | m.p. 81.4-88.5°C |
| 341 | CH₃ | NH(4-C₂H₅-Ph) | m.p. 95.1-96.2°C |
| 342 | CH₃ | NH(2-COOC₂H₅-Ph) | m.p. 63°C |
| 343 | CH₃ | NH(3-COOC₂H₅-Ph) | paste |
| 344 | CH₃ | NH(4-COOC₃H₇-i-Ph) | m.p. 125.2-127.5°C |
| 345 | CH₃ | NH(2-CONH₂-Ph) | m.p. 222-223°C |
| 346 | CH₃ | NH(3-CONH₂-Ph) | m.p. 232°C |
| 347 | CH₃ | NH(4-CONH₂-Ph) | m.p. 244.8-248.2°C |
| 348 | CH₃ | NH(2-CONHCH₃-Ph) | m.p. 162°C |
| 349 | CH₃ | NH(3-CONHCH₃-Ph) | m.p. 181°C |
| 350 | CH₃ | NH(4-CONHCH₃-Ph) | m.p. 196.3-198.1°C |
| 351 | CH₃ | NH(2-CONHC₂H₅-Ph) | m.p. 166°C |
| 352 | CH₃ | NH(3-CONHC₂H₅-Ph) | m.p. 155°C |
| 353 | CH₃ | NH(4-CONHC₂H₅-Ph) | m.p. 190.8-193.4°C |
| 354 | CH₃ | NH(2-CONHC₃H₇-n-Ph) | m.p. 165°C |
| 355 | CH₃ | NH(3-CONHC₃H₇-n-Ph) | paste |
| 356 | CH₃ | NH(4-CONHC₃H₇-n-Ph) | m.p. 174.7-175.9°C |
| 357 | CH₃ | NH(2-CONHC₃H₇-i-Ph) | m.p. 177°C |
| 358 | CH₃ | NH(3-CONHC₃H₇-i-Ph) | m.p. 158°C |
| 359 | CH₃ | NH(4-CONHC₃H₇-i-Ph) | m.p. 223.7-225.9°C |
| 360 | CH₃ | NH(3-CONHC₄H₉-n-Ph) | paste |
| 361 | CH₃ | NH(4-CONHC₄H₉-n-Ph) | m.p. 157.4-159.3°C |
| 362 | CH₃ | NH(3-CONHC₄H₉-t-Ph) | paste |
| 363 | CH₃ | NH(4-CONHC₄H₉-t-Ph) | m.p. 229.4-231.1°C |
| 364 | CH₃ | NH(2-CONHPh-Ph) | m.p. 172-174°C |
| 365 | CH₃ | NH(4-CONHPh-Ph) | m.p. 258.2-249.1°C |
| 366 | CH₃ | NH(2-CON(CH₃)₂-Ph) | m.p. 153°C |
| 367 | CH₃ | NH(3-CON(CH₃)₂-Ph) | m.p. 143°C |
| 368 | CH₃ | NH(4-CON(CH₃)₂-Ph) | m.p. 176.8-178.6°C |
| 369 | CH₃ | NH(3-CON(C₂H₅)₂-Ph) | paste |
| 370 | CH₃ | NH(4-CON(C₂H₅)₂-Ph) | m.p. 157.4-160.2°C |
| 371 | CH₃ | NH(3-CONHC₆H₁₃-n-Ph) | paste |
| 372 | CH₃ | NH(3-CONH(4-C1-Ph)-Ph) | paste |
| 373 | CH₃ | NH(3-CO-Q₁₀-Ph) | paste |
| 374 | CH₃ | NH(2-COOH-Ph) | m.p. 223°C |
| -375 | CH₃ | NH(3-COOH-Ph) | m.p. 237°C |
| 376 | CH₃ | NH(2-C₃H₇-n-Ph) | m.p. 76-78°C |
| 377 | CH₃ | NH(4-C₃H₇-n-Ph) | m.p. 76-82°C |
| 378 | CH₃ | NH(2-C₃H₇-i-Ph) | m.p. 116-118°C |
| 379 | CH₃ | NH(4-C₃H₇-i-Ph) | m.p. 115-117°C |
| 380 | CH₃ | NH(4-C₄H₉-n-Ph) | paste |
| 381 | CH₃ | NH(4-C₄H₉-sec-Ph) | m.p. 89-91°C |
| 382 | CH₃ | NH(2-C₄H₉-t-Ph) | m.p. 114-115°C |
| 383 | CH₃ | NH(4-C₄H₉-t-Ph) | paste |
| 384 | CH₃ | NH(4-C₈H₁₇-n-Ph) | m.p. 51-52°C |
| 385 | CH₃ | NH(2-Br-Ph) | m.p. 139.8-142.1°C |
| 386 | CH₃ | NH(3-Br-Ph) | m.p. 144.4-146.3°C |
| 387 | CH₃ | NH(4-Br-Ph) | m.p. 122.9-124.4°C |
| 388 | CH₃ | NH(2,6-Cl₂-Ph) | m.p. 168°C |
| 389 | CH₃ | NH(2,3-Cl₂-Ph) | m.p. 151°C |
| 390 | CH₃ | NH(2-C₂H₅-Ph) | m.p. 99.5-101.O°C |
| 391 | CH₃ | NH(3,5-(CH₃)₂-Ph) | m.p. 135°C |
| 392 | CH₃ | NH(2,3-(CH₃)₂-Ph) | m.p. 124°C |
| 393 | CH₃ | NH(3,4-(CH₃)₂-Ph) | m.p. 116.8-118.2°C |
| 394 | CH₃ | NH(2,5-(CH₃)₂-Ph) | m.p. 140°C |
| 395 | CH₃ | NH(2,4-(OCH₃)₂-Ph) | m.p. 136°C |
| 396 | CH₃ | NH(2,5-(OCH₃)₂-Ph) | m.p. 142°C |
| 397 | CH₃ | NH(3,4-(OCH₃)₂-Ph) | m.p. 133°C |
| 398 | CH₃ | NH(3,5-(OCH₃)₂-Ph) | m.p. 132°C |
| 399 | CH₃ | NH(4-OC₂H₅-Ph) | m.p. 105.9-106.7°C |
| 400 | CH₃ | NH(2-NH₂-Ph) | |
| 401 | CH₃ | NH(3-NH₂-Ph) | m.p. 168°C |
| 402 | CH₃ | NH(4-NH₂-Ph) | m.p. 181°C |
| 403 | CH₃ | NH(4-CH₃CONH-Ph) | m.p. 206°C |
| 404 | CH₃ | NH(4-PhCONH-Ph) | m.p. 272°C |
| 405 | CH₃ | NH(4-(4-CH₃-Ph)SO₂NHPh) | m.p. 179°C |
| 406 | CH₃ | NH(2-NHCOCH₃-Ph) | |
| 407 | CH₃ | NH(3-NHCOCH₃-Ph) | |
| 408 | CH₃ | NH(4-NHCOC₂H₅-Ph) | |
| 409 | CH₃ | NH( 4-NHCOC)H₇-i-Ph) | |
| 410 | CH₃ | NH(2-NHCOC₄H₉-t-Ph) | |
| 411 | CH₃ | NH(3-NHCOC₄H₉-t-Ph) | |
| 412 | CH₃ | NH(4-NHCOC₄H₉-t-Ph) | |
| 413 | CH₃ | NH(4-NHCOOCH₃-Ph) | |
| 414 | CH₃ | NH(2-NHCOOC₂H₅-Ph) | |
| 415 | CH₃ | NH(3-NHCOOC₂H₅-Ph) | |
| 416 | CH₃ | NH(4-NHCOOC₂H₅-Ph) | |
| 417 | CH₃ | NH(2-NHCOPh-Ph) | |
| 418 | CH₃ | NH(3-NHCOPh-Ph) | |
| 419 | CH₃ | NH(4-NHCO(6-C1-Q₁)-Ph) | |
| 420 | CH₃ | NH(2-NHCO(4-CH₃-Q₈)-Ph) | m.p. 189°C |
| 421 | CH₃ | NH(4-NHCO(4-CH₃-Q₈)-Ph) | m.p. 260°C |
| 422 | CH₃ | NH(4-NHCONHC₂H₅-Ph) | |
| 423 | CH₃ | NH(2-NHSO₂CH₃-Ph) | |
| 424 | CH₃ | NH(3-NHSO₂CH₃-Ph) | |
| 425 | CH₃ | NH(4-NHSO₂CH₃-Ph) | |
| 426 | CH₃ | NH(2-NO₂-Ph) | |
| 427 | CH₃ | NH(3-NO₂-Ph) | m.p. 140°C |
| 428 | CH₃ | NH(2-OH-Ph) | |
| 429 | CH₃ | NH(3-OH-Ph) | |
| 430 | CH₃ | NH(4-OH-Ph) | |
| 431 | CH₃ | NH(2-O-COCH₃-Ph) | |
| 432 | CH₃ | NH(3-0-COCH₃-Ph) | |
| 433 | CH₃ | NH(4-O-COCH₃-Ph) | |
| 434 | CH₃ | NH(2-O-COC₄H₉-t-Ph) | |
| 435 | CH₃ | NH(3-0-COC₄H₉-t-Ph) | |
| 436 | CH₃ | NH(4-O-COC₄H₉-t-Ph) | |
| 437 | CH₃ | NH(3-O-COPh-Ph) | |
| 438 | CH₃ | NH(4-O-COPh-Ph) | |
| 439 | CH₃ | NH(2-O-CO(4-CH₃-Q₈)-Ph | m.p. 112°C |
| 440 | CH₃ | NH(3-O-CO(4-CH₃-Q₈)-Ph | m.p. 152°C |
| 441 | CH₃ | NH(4-O-CO(4-CH₃-Q₈)-Ph | m.p. 167°C |
| 442 | CH₃ | NH(2-O-COOC₂H₅-Ph) | |
| 443 | CH₃ | NH(3-O-COOC₂H₅-Ph) | |
| 444 | CH₃ | NH(4-O-COOC₂H₅-Ph) | |
| 445 | CH₃ | NH(4-O-COOC₂H₅-Ph) | |
| 446 | CH₃ | NH(4-CH₃SO₂-Ph) | |
| 447 | CH₃ | NH(4-CF₃SO₂-Ph) | |
| 448 | CH₃ | NH(5-CH₃-Q₁₈) | m.p. 156°C |
| 449 | CH₃ | NH(2-CH₃-Q₁₉) | m.p. 189°C |
| 450 | CH₃ | NH(4,6-(CH₃)₂-Q₁₁) | paste |
| 451 | CH₃ | NHCH₂CONH(4-Cl-Ph) | m.p. 211°C |
| 452 | CH₃ | NHCH₂CONH(4-CH₃-Ph) | m.p. 212°C |
| 953 | CH₃ | NHCH₂CONH(4-OCH₃-Ph) | m.p. 188°C |
| 454 | CH₃ | NHCH₂CONHCH₂(4-CH₃-Ph) | paste |
| 455 | CH₃ | Q₂₀-(4-CO(4-CH₃-Q₈)) | m.p. 234°C |
| 456 | CH₃ | NH-(5-Br-Q₁₁) | |
| 457 | CH₃ | NH-(4,6-(Cl)₂-Q₁₁) | |
| 458 | CH₃ | NH-(4,6-(CH₃)₂-Q₁₁) | m.p. 153°C |
| 459 | CH₃ | NH-Q₁ | |
| 460 | CH₃ | NH-Q₂₁ | |
| 461 | CH₃ | NH-(4-CH₃-Q₂₁) | |
| 462 | CH₃ | NH-(4-Ph-Q₂₁) | |
| 463 | CH₃ | NH-(5-CF₃-Q₁₉) | |
| 464 | CH₃ | NH-(5-SC₂H₅-Q₁₉) | |
| 465 | CH₃ | NH-(3-Cl-5-CF₃-Q₂) | |
| 466 | CH₃ | NH-(4,6-(CH₃)₂-Q₂) | |
| 467 | CH₃ | NH-Q₂₂ | |
| 468 | CH₃ | NH(CH₂)₂NH-CO(4-CH₃-Q₈) | |
| 469 | CH₃ | NH(CH2)₃NH-CO(4-CH₃-Q₈) | |
| 470 | CH₃ | NH(CH₂)₆NH-CO(4-CH₃-Q₈) | |
| 471 | CH₃ | NH(CH₂)₁₂NH-CO(4-CH₃-Q₈) | |
| 472 | CH₃ | NH(Ph)-NH₂ | |
| 489 | CH₃ | NBOC₂H₅ | |
| 490 | CH₃ | NHOC₃H₇-n | |
| 491 | CH₃ | NHOC₃H₇-i | |
| 492 | CH₃ | NBOC₄H₉-n | |
| 493 | CH₃ | NHOC₄H₉-t | |
| 494 | CH₃ | N(CH₃)OCH₃ | |
| 495 | CH₃ | NHOCH₂CH=CH₂ | |

| | | | |
|---|---|---|---|
| Note: "cyclo" means a cyclic C₆H₁₁ group. | | | |

Table 2 shows ¹H-NMR data of the 1,2,3-thiadiazole derivatives having a physical property expressed by the word "oil", "paste" or "NMR" in Table 1.

**Table 2**

| No | ¹H-NMR [CDCl₃/TMS, δ value (ppm) |
|---|---|
| 96 | 1.595 (s, 1H), 1.757 (s, 6H), 2.910 (s, 3H), 5.98 (br, 1H). |
| 97 | 1.305 (d, 2H), 1.85 (br, 1H), 2.903 (s, 3H), 3.735 (m, 2H), 4.25 (m, 1H), 6.42 (br, 1H). |
| 102 | 1.00 (t, 6H), 2.30 (m, 1H), 2.950 (s, 3H), 3.800 (s, 3H), 4.740 (m, 1H), 6.42 (br, 1H). |
| 114 | 1.342 (d, 6H), 2.948 (s, 3H), 4.567 (sept, 1H), 6.743 (d, 1H), 7.011 (d, 1H), 7.23-7.28 (m, 2H), 7.659 (s, 1H). |
| 118 | 1.2695 (d, 6H), 2.9355 (sept, 1H), 2.9868 (s, 3H), 7.1260 (d, 1H), 7.32-7.40 (m, 3H), 7.47 (br, s, 1H) . |
| 140 | 2.815 (s, 3H), 3.482 (s, 3H), 7.11 (m, 2H), 7.36 (m, 3H). |
| 144 | 2.792 (s, 3H), 3.25 (m, 1H), 3.822 (s, 3H), 5.05 (m, 1H), 6.32 (br, 1H), 7.05-7.40 (m, 5H). |
| 150 | 1.26 (t, 3H), 2.90 (s, 3H), 3.74 (q, 2H), 6.11 (d, 1H), 8.03 (br, d, 1H). |
| 151 | 2.637 (t, 1H), 2.971 (s, 1H), 4.425 (m, 2H), 6.374 (d, 1H), 7.13 (br, d, 1H). |
| 154 | 1.01 (m, 3H), 1.39 (q, 3H), 1.65 (m, 2H), 2.94 (s, 3H), 3.10 (m, 1H), 6.07 (m, 1H), 7.05 (br, t, 1H). |
| 193 | 1.487 (d, 6H), 3.73 (sept, 1H), 4.365 (d, 2H), 6.78 (br, s, 1H) |
| 213 | 1.345 (d, 6H), 4.568 (s, 1H), 6.70-7.30 (m, 4H), 7.92 (br, s, 1H). |
| 227 | 1.20-1.40 (m, 10H), 2.53 (m, 1H), 2.89 (m, 1H), 7.05-7.50 (m, 4H), 8.30 (br, s, 1H). |
| 228 | 1.20-1.40 (m, 10H), 2.50 (m, 1H), 4.55 (m, 1H), 6.72 (q, 1H), 7.0-7.3 (m, 3H), 8.30 (br, s, 1H). |
| 242 | 0.98 (t, 3H), 1.49 (m, 2H), 1.84 (m, 2H), 4.50 (t, 2H), 4.63 (d, 2H), 7.3-7.6 (m, 5H), 10.23 (br, s, 1H). |
| 296 | 0.939 (t, 3H), 1.45 (m, 2H), 1.65 (m, 2H), 2.970 (s, 3H), 3.82 (m, 2H), 5.75 (d, 1H), 6.48 (d, 1H). |
| 343 | 1.38 (t, 3H), 2.96 (s, 3H), 4.32 (dd, 2H), 7.47 (t, 1H), 7.85 (d, 1H), 8.00-8.05 (br, 2H), 8.21 (br, 1H). |
| 355 | 0.86 (t, 3H), 1.46 (m, 2H), 2.87 (s, 3H), 3.07 (dd, 2H), 6.43 (m, 1H), 7.39 (d, 2H), 7.97 (br, 1H), 8.10 (m, 1H), 9.75 (br, 1H). |
| 360 | 0.92 (t, 3H), 1.36 (m, 2H), 1.51 (m, 2H), 3.21 (dd, 2H), 6.21 (br, 1H), 7.44 (d, 2H), 7.98 (s, 1H), 8.08 (br, 1H), 9.19 (br, 1H). |
| 362 | 1.36 (s, 9H), 2.84 (s, 3H), 6.05 (s, 1H), 7.34 (m, 2H), 7.81 (s, 1H), 7.90 (m, 1H), 9.41 (br, 1H). |
| 369 | 1.03-1.21 (m, 6H), 3.19 (dd, 2H), 3.41 (dd, 2H), 6.87 (d, 1H), 7.24 (m, 2H), 7.73 (d, 1H), 10.21 (br, 1H). |
| 371 | 0.89 (t, 3H), 1.20-1.70 (m, 8H), 3.24 (dd, 2H), 6.21 (br, 1H), 7.40-7.50 (m, 1H), 7.99 (s, 1H), 8.05 (br, 1H), 9.01 (br, 1H). |
| 372 | 2.95 (s, 3H), 7.25-7.95 (m, 7H), 8.06-8.14 (br, 2H), 8.56 (br, 1H). |
| 373 | 2.89 (s, 3H), 3.4-3.8 (br, 8H), 7.02 (d, 1H), 7.35 (t, 1H), 7.47 (br, 1H), 7.87 (d, 1H), 9.53 (br, 1H). |
| 380 | 0.93 (t, 3H), 1.37 (m, 2H), 1.59 (m, 2H), 2.61 (t, 2H), 2.97 (s, 1H), 7.22 (d, 2H), 7.45 (d, 2H), 7.53 (br, 1H). |
| 383 | 1.32 (s, 9H), 2.96 (s, 3H), 7.34 (d, 2H), 7.49 (d, 2H), 7.88 (br, 1H). |
| 450 | 2.42 (s, 6H), 2.90 (s, 3H), 6.82 (s, 1H), 8.6-9.5 (br, 1H). |
| 454 | 2.28 (s, 3H), 2.81 (s, 3H), 3.92 (d, 2H), 4.26 (d, 2H), 7.12 (m, 4H), 8.51 (br, 1H), 9.08 (br, 1H). |

The 1,2,3-thiadiazole derivatives of the general formula (I) or salts thereof according to the present invention are useful for agricultural and horticultural disease control. For example, the compounds listed in Table 1 are very effective in controlling various diseases, for instance, rice blast (Pyricularia oryzae), rice sheath blight (Rhizoctonia solani), rice helminthosporium leaf spot (Cochliobolus miyabeanus), powdery mildew of various host plants, such as powdery mildew of barley and wheat (Erysiphe graminis), oats crown rust (Puccinia coronata), rust of other plants, tomato late blight (Phytophthora infestans), late blight or Phytophthora rots of other plants, downy mildew of various plants, such as cucumber downy mildew (Pseudoperonospora cubensis) and grape downy mildew (Plasmopara viticola), apple scab (Venturia inaequalis), apple alternaria leaf spot (Alternaria mali), pear black spot (Alternaria kikuchiana), citrus melanose (Diaporthe citri), cucumber bacterial blight (Pseudomonas syringae pv. lachrymans), tomato bacterial wilt (Pseudomonas solanacearum), cabbage black rot (Xanthomonas campestris), citrus canker (Xanthomonas citri (Hasse) Dowson), rice bacterial leaf blight (Xanthomonas oryzae), cabbage bacterial soft rot (Erwinia carotovora), and tobacco mosaic (Tobacco mosaic virus).

The agricultural and horticultural disease controller of the present invention is markedly effective in controlling the above-exemplified diseases which damage paddy field crops, upland crops, fruit trees, vegetables, other crops, flowers and ornamental plants, and the like. Therefore, the desired effects of the agricultural and horticultural disease controller of the present invention can be obtained by applying the disease controller to the paddy field water, stalks and leaves of fruit trees, vegetables, other crops, flowers and ornamental plants, soil, etc., at a season at which the diseases are expected to occur, before their occurrence or at the time when their occurrence is confirmed.

In general, the agricultural and horticultural disease controller of the present invention is used after being prepared into a conveniently usable form according to an ordinary manner for preparation of agrochemicals.

That is, the 1,2,3-thiadiazole derivative of the general formula (I) or salt thereof according to the present invention and, optionally, an adjuvant are blended with a suitable inert carrier in a proper proportion and prepared into a suitable preparation form such as a suspension, emulsifiable concentrate, soluble concentrate, wettable powder, granules, dust or tablets through dissolution, dispersion, suspension, mixing, impregnation, adsorption or sticking.

The inert carrier used in the present invention may be either solid or liquid. As the solid carrier, there can be exemplified soybean flour, cereal flour, wood flour, bark flour, saw dust, powdered tobacco stalks, powdered walnut shells, bran, powdered cellulose, extraction residue of vegetables, powdered synthetic polymers or resins, clays (e.g. kaolin, bentonite, and acid clay), talcs (e.g. talc and pyrophyllite), silica powders or flakes (e.g. diatomaceous earth, silica sand, mica and white carbon, i.e. synthetic, high-dispersion silicic acid, also called finely divided hydrated silica or hydrated silicic acid, some of commercially available products contain calcium silicate as the major component), activated carbon, powdered sulfur, powdered pumice, calcined diatomaceous earth, ground brick, fly ash, sand, calcium carbonate powder, calcium phosphate powder and other inorganic or mineral powders, chemical fertilizers (e.g. ammonium sulfate, ammonium phosphate, ammonium nitrate, urea and ammonium chloride), and compost. These carriers may be used alone or as a mixture thereof.

The liquid carrier is that which itself has solubility or which is without such solubility but is capable of dispersing an active ingredient with the aid of an adjuvant. The following are typical examples of the liquid carrier and can be used alone or as a mixture thereof. Water; alcohols such as methanol, ethanol, isopropanol, butanol and ethylene glycol; ketones such as acetone, methyl ethyl ketone, methyl isobutyl ketone, diisobutyl ketone and cyclohexanone; ethers such as ethyl ether, dioxane, Cellosolve, dipropyl ether and tetrahydrofuran; aliphatic hydrocarbons such as kerosene and mineral oils; aromatic hydrocarbons such as benzene, toluene, xylene, solvent naphtha and alkylnaphthalenes; halogenated hydrocarbons such as dichloroethane, chloroform, carbon tetrachloride and chlorobenzene; esters such as ethyl acetate, diisopropyl phthalate, dibutyl phthalate and dioctyl phthalate; amides such as dimethylformamide, diethylformamide and dimethylacetamide; nitriles such as acetonitrile; and dimethyl sulfoxide.

The following are typical examples of the adjuvant, which are used depending upon purposes and used alone or in combination in some cases, or need not to be used at all.

To emulsify, disperse, dissolve and/or wet an active ingredient, a surfactant is used. As the surfactant, there can be exemplified polyoxyethylene alkyl ethers, polyoxyethylene alkylaryl ethers, polyoxyethylene higher fatty acid esters, polyoxyethylene resinates, polyoxyethylene sorbitan monolaurate, polyoxyethylene sorbitan monooleate, alkylarylsulfonates, naphthalenesulfonic acid condensation products, ligninsulfonates and higher alcohol sulfate esters.

Further, to stabilize the dispersion of an active ingredient, tackify it and/or bind it, there may be used adjuvants such as casein, gelatin, starch, methyl cellulose, carboxymethyl cellulose, gum arabic, polyvinyl alcohols, turpentine, bran oil, bentonite and ligninsulfonates.

To improve the flowability of a solid product, there may be used adjuvants such as waxes, stearates and alkyl phosphates.

Adjuvants such as naphthalenesulfonic acid condensation products and polycondensates of phosphates may be used as a peptizer for dispersible products.

Adjuvants such as silicon oils may also be used as a defoaming agent.

The content of the active ingredient may be varied as required. In dusts or granules, the suitable content thereof is from 0.01 to 50% by weight. In emulsifiable concentrates or flowable wettable powders, it is also from 0.01 to 50% by weight.

The present inventive agricultural and horticultural disease controller containing the 1,2,3-thiadiazole derivative of the general formula (I) or a salt thereof as an active ingredient is used to control various diseases in the following manner. That is, it is applied to a crop on which the diseases are expected to occur, or a site where the occurrence of the diseases is undesirable, as it is or after being properly diluted with or suspended in water or the like, in an amount effective for control of the diseases. For example, to control the diseases of paddy rice, said disease controller can be used by a method such as submerged application to a regular paddy field, application to a rice nursery bed, dressing of seeds for direct sowing on flooded paddy field, or seed disinfection.

The applying dosage of the present inventive agricultural and horticultural disease controller containing the 1,2,3-thiadiazole derivative of the general formula (I) or a salt thereof as an active ingredient is varied depending upon various factors such as a purpose, diseases to be controlled, a growth state of a plant, tendency of disease occurrence, weather, environmental conditions, a preparation form, an application method, an application site and application time. It may be properly chosen in the range of 0.1 g to 10 kg (in terms of the active ingredient) per 10 ares depending upon purposes.

The present inventive agricultural and horticultural disease controller containing the 1,2,3-thiadiazole derivative of the general formula (I) or a salt thereof as an active ingredient may be used in admixture with other agricultural and horticultural disease controllers in order to expand both spectrum of controllable diseases and the period of time when effective applications are possible or to reduce the dosage.

Typical examples and test examples of the present invention are described below but they should not be construed as limiting the scope of the invention.

In the examples, parts are all by weight.

### Example 1

| | |
|---|---|
| Each compound listed in Table 1 | 50 parts |
| Xylene | 40 parts |
| Mixture of polyoxyethylene nonylphenyl ether and calcium alkylbenzenesulfonate | 10 parts |

An emulsifiable concentrate was prepared by mixing uniformly the above ingredients to effect dissolution.

### Example 2

| | |
|---|---|
| Each compound listed in Table 1 | 3 parts |
| Clay powder | 82 parts |
| Diatomaceous earth powder | 15 parts |

A dust was prepared by mixing uniformly and grinding the above ingredients.

### Example 3

| | |
|---|---|
| Each compound listed in Table 1 | 5 parts |
| Mixed powder of bentonite and clay | 90 parts |
| Calcium lignin sulfonate | 5 parts |

Granules were prepared by mixing the above ingredients uniformly, and kneading the resulting mixture together with a suitable amount of water, followed by granulation and drying.

### Example 4

| | |
|---|---|
| Each compound listed in Table 1 | 20 parts |
| Mixture of kaolin and synthetic, high-dispersion silicic acid | 75 parts |
| Mixture of polyoxyethylene nonylphenyl ether and calcium alkylbenzenesulfonate | 5 parts |

A wettable powder was prepared by mixing uniformly and grinding the above ingredients.

### Test Example 1

### Controlling effect on rice blast by submerged application

Rice plants at the 5 to 6 leaf stage cultivated in a 1/10000-are pot were subjected to submerged application of a chemical containing each compound listed in Table 1 as an active ingredient, in a dosage of 200 g/10 a in terms of the active ingredient. After standing in a greenhouse for 1 week, the plants were inoculated with a suspension of spores of blast fungus (Pyricularia oryzae) by spraying.

After the inoculation, the plants were allowed to stand in a moist chamber for 1 day and then a greenhouse for 6 days to cause the disease sufficiently. Then, lesions in each leaf were counted and then compared with those on the untreated plot, and the controlling degree was calculated, whereby the effect was judged according to the following criterion.

| Effect | Controlling degree (%) |
|---|---|
| A | 100 - 95 |
| B | 94 - 85 |
| C | 84 - 60 |
| D | 59 - 0 |

As a result of the above test, the compounds listed in Table 1 were found to have a marked blast-controlling activity. Of these compounds, the following were rated C or higher: compound Nos. 1 to 6, 85 to 89, 92 to 95, 97 to 100, 103 to 131, 133 to 136, 138 to 140, 142, 144 to 156, 162, 173 to 192, 194 211 to 216, 227 to 228, 240, 253, 288 to 296, 342 to 346, 348, 351, 354 to 357, 361, 362, 366, 369, 372, 374 and 375. In particular, the following were rated A, namely, the following had an excellent blast-controlling activity: compound Nos. 5 92, 93, 99, 103, 104 to 121, 123 to 125, 128, 130, 131, 138, 145, 146, 149, 152, 153, 156, 173, 174, 183, 192, 194, 213, 215 to 216,

227 to 228, 253, 288 to 290, 294, 343, 344, 346, 355, 356, 362, 372, 374 and 375.

### Test Example 2

### Controlling effect on barley powdery mildew

Barley plants at the 3.5 leaf stage cultivated in a pot were applied with a spray mix containing each compound listed in Table 1 as an active ingredient at the concentration of 200 ppm. After standing thus treated plants in a greenhouse for 1 week, the plants were inoculated with spores of powdery mildew fungus (Erysiphe graminis f. sp. hordei)

After the inoculation, the plants were allowed to stand in a greenhouse for 1 week to cause the disease sufficiently. Then, lesions in each leaf were counted and then compared with those on the untreated plot, and the controlling degree was calculated, whereby the effect was judged according to the following criterion.

| Effect | Controlling degree (%) |
|---|---|
| A | 100 - 80 |
| B | 79 - 60 |
| C | 59 - 0 |

As a result of the above test, the compounds listed in Table 1 were found to have a marked powdery mildew-controlling activity. Of these compounds, the following were rated B or higher: compound Nos. 104 to 107, 111 to 113, 115 to 117, 122 to 124, 128, 130, 139, 145, 193, 194 and 215. In particular, the following were rated A: compound Nos. 104 to 107, 111 to 113, 115 to 117, 122 to 124, 128, 130, 139, 145, 193 and 215.

### Referential Production Example

### Production of N-isopropyl-5-(N'-isopropylcarbamoyl)-1,2,3-thiadiazol-4-ylacetamide (compound No. 252)

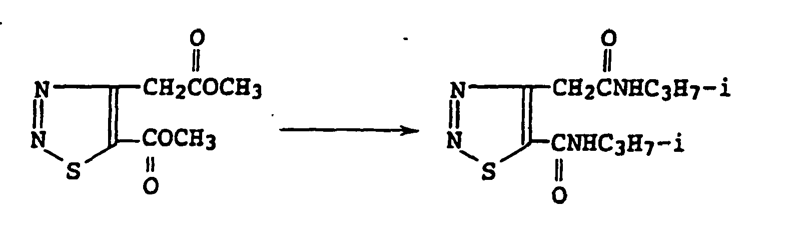

In dimethyl sulfoxide was suspended 0.1 g of sodium hydride, after which 1 g of isopropylamine was added to the suspension, followed by ice-cooling. Then, 0.5 g of methyl 5-methoxycarbonyl-1,2,3-thiadiazol-4-ylacetate was added and the resulting mixture was stirred at room temperature and allowed to stand for 24 hours.

After completion of the reaction, the reaction mixture was poured into ice water and the desired compound was extracted with ethyl acetate. The extracted solution was washed with water, dried over anhydrous sodium sulfate, and then distilled under reduced pressure to remove the solvent. The crude product thus obtained was purified by a column chromatography to obtain 0.23 g of the desired compound.
Physical property: m.p. 146 - 156°C.
Yield: 37%.

## Claims

1. Use of an agricultural and horticultural disease controller comprising as an active ingredient a 1,2,3-thiadiazole derivative represented by the general formula (I), or a salt thereof 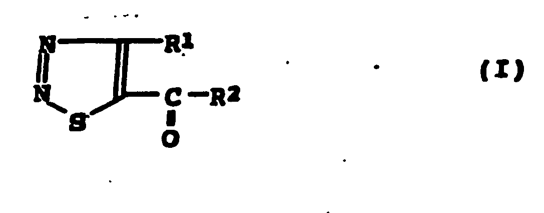 wherein R¹ is ① a hydrogen atom, ② a (C₁-C₁₂)alkyl group, ③ a halo-(C₁-C₁₂)alkyl group, ④ a (C₂-C₁₂)alkenyl group, ⑤ a halo (C₂-C₁₂)alkenyl group, ⑥ a (C₂-C₁₂)alkynyl group, ⑦ a halo(C₂-C₁₂)alkynyl group, ⑧ a (C₃-C₆)cycloalkyl group, ⑨ an unsubstituted phenyl group, ⑧ a substituted phenyl group having one or more substituents which may be the same or different and are selected from the group consisting of halogen atoms, nitro group, cyano group, (C₁-C₆)alkyl groups, halo(C₁-C₆)alkyl groups, (C₁-C₆)alkoxy groups, halo(C₁-C₆)alkoxy groups, (C₁-C₆)alkylthio groups, halo(C₁-C₆)alkylthio groups, (C₂-C₆)alkenyl groups and (C₂-C₆)alkynyl groups, ⑪ a group represented by the formula: 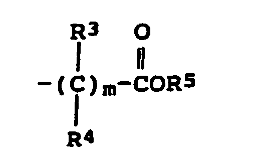 wherein R³ and R⁴, which may be the same or different, are hydrogen atoms, (C₁-C₁₂)alkyl groups or halo-(C₂-C₁₂)alkyl groups, m is zero or an integer of 1 to 6, and R⁵ is a hydrogen atom; a (C₁-C₁₂)alkyl group; a halo(C₁-C₁₂)alkyl group; a (C₂-C₁₂)alkenyl group; a (C₂-C₁₂)alkynyl group; an unsubstituted phenyl group; a substituted phenyl group having one or more substituents which may be the same or different and are selected from the group consisting of halogen atoms, nitro group, cyano group, (C₁-C₆)alkyl groups, halo(C₁-C₆)alkyl groups, (C₁-C₆)alkoxy groups, halo(C₁-C₆)alkoxy groups, (C₁-C₆)alkylthio groups, halo(C₁-C₆)alkylthio groups, (C₂-C₆)alkenyl groups and (C₂-C₆)alkynyl groups; an unsubstituted phenyl(C₁-C₆)alkyl group; or a substituted phenyl(C₁-C₆)alkyl group having on the ring one or more substituents which may be the same or different and are selected from the group consisting of halogen atoms, nitro group, cyano group, (C₁-C₆)alkyl groups, halo(C₁-C₆)alkyl groups, (C₁-C₆)alkoxy groups, halo(C₁-C₆)alkoxy groups, (C₁-C₆)alkylthio groups, halo(C₁-C₆)alkylthio groups, (C₂-C₆)alkenyl groups and (C₂-C₆)alkynyl groups , ⑫ a group represented by the formula: 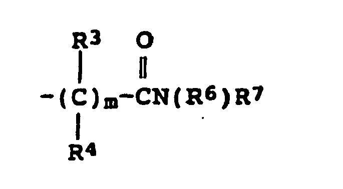 wherein R³, R⁴ and m are as defined above, and R⁶ and R⁷, which may be the same or different, are hydrogen atoms; (C₁-C₁₂)alkyl groups; halo(C₁-C₁₂)alkyl groups; (C₂-C₁₂)alkenyl groups; (C₂-C₁₂)alkynyl groups; (C₁-C₁₂)alkoxy(C₁-C₆)alkyl groups; (C₁-C₁₂)alkylthio-(C₁-C₆)alkyl groups; cyano(C₁-C₁₂)alkyl groups; unsubstituted phenyl groups; substituted phenyl groups having one or more substituents which may be the same or different and are selected from the group consisting of halogen atoms, nitro group, cyano group, (C₁-C₆)alkyl groups, halo(C₁-C₆)alkyl groups, (C₁-C₆)alkoxy groups, halo(C₁-C₆)alkoxy groups, (C₁-C₆)alkylthio groups, halo(C₁-C₆)alkylthio groups, (C₂-C₆)alkenyl groups and (C₂-C₆)alkynyl groups; unsubstituted phenyl(C₁-C₆)alkyl groups; or substituted phenyl(C₁-C₆)alkyl groups having on the ring one or more substituents which may be the same or different and are selected from the group consisting of halogen atoms, nitro group, cyano group, (C₁-C₆)alkyl groups, halo(C₁-C₆)alkyl groups, (C₁-C₆)alkoxy groups, halo(C₁-C₆)alkoxy groups, (C₁-C₆)alkylthio groups, halo(C₁-C₆)alkylthio groups, (C₂-C₆)alkenyl groups and (C₂-C₆)alkynyl groups; R⁶ and R⁷ being able to be taken together to represent a (C₄-C₆)alkylene group which may contain an oxygen atom between adjacent carbon atoms of the carbon chain , ⑬ a group represented by the formula: 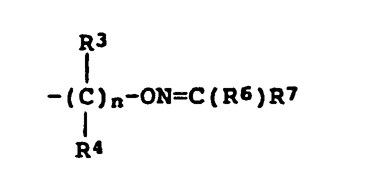 wherein R³, R⁴, R⁶ and R⁷ are as defined above, and n is an integer of 1 to 6 , or ⑭ a group represented by the formula: 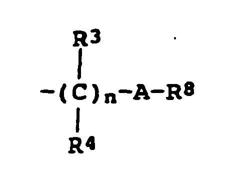 wherein R³, R⁴ and n are as defined above, A is
-O-, 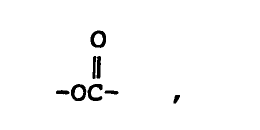 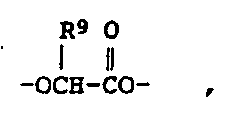 wherein R⁹ is a hydrogen atom, a (C₁-C₁₂)alkyl group or a halo(C₁-C₁₂)alkyl group),
-S- ,
-SO- ,
-SO₂- , 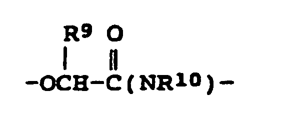 wherein R⁹ is as defined above, and R¹⁰ is a hydrogen atom, a (C₁-C₁₂)alkyl group or a halo(C₁-C₁₂)alkyl group, R⁹ and R¹⁰ being able to be taken together to represent a (C₄-C₆)alkylene group which may contain, between adjacent carbon atoms of the carbon chain, an oxygen atom, a sulfur atom or
N-R¹¹
wherein R¹¹ is a hydrogen atom, a (C₁-C₁₂)alkyl group or a halo(C₁-C₁₂)alkyl group, or
-N(R¹⁰)-
wherein R¹⁰ is as defined above , and R⁸ is a hydrogen atom; a (C₁-C₁₂)alkyl group; a halo(C₁-C₁₂)alkyl group; a (C₂-C₁₂)alkenyl group; a (C₂-C₁₂)alkynyl group; a (C₁-C₁₂)alkoxy(C₁-C₁₂)alkyl group; a (C₁-C₁₂)alkylthio-(C₁-C₁₂)alkyl group; a cyano(C₁-C₁₂)alkyl group; an unsubstituted phenyl group; a substituted phenyl group having one or more substituents which may be the same or different and are selected from the group consisting of halogen atoms, nitro group, cyano group, (C₁-C₆)alkyl groups, halo(C₁-C₆)alkyl groups, (C₁-C₆)alkoxy groups, halo(C₁-C₆)alkoxy groups, (C₁-C₆)alkylthio groups, halo(C₁-C₆)alkylthio groups, (C₂-C₆)alkenyl groups and (C₂-C₆)alkynyl groups; an unsubstituted phenyl(C₁-C₆)-alkyl group; a substituted phenyl(C₁-C₆)alkyl group having on the ring one or more substituents which may be the same or different and are selected from the group consisting of halogen atoms, nitro group, cyano group, (C₁-C₆)alkyl groups, halo(C₁-C₆)alkyl groups, (C₁-C₆)-alkoxy groups, halo(C₁-C₆)alkoxy groups, (C₁-C₆)alkyl-thio groups, halo(C₁-C₆)alkylthio groups, (C₂-C₆)alkenyl groups and (C₂-C₆)alkynyl groups; an unsubstituted 5- or 6-membered heterocyclic ring having one or more heteroatoms which may be the same or different and are selected from the group consisting of oxygen atom, sulfur atom and nitrogen atom; or a substituted 5- or 6-membered heterocyclic ring having one or more heteroatoms which may be the same or different and are selected from the group consisting of oxygen atom, sulfur atom and nitrogen atom, said substituted heterocyclic ring having one or more substituents which may be the same or different and are selected from the group consisting of halogen atoms, nitro group, cyano group, (C₁-C₆)alkyl groups, halo(C₁-C₆)alkyl groups, (C₁-C₆)-alkoxy groups, halo(C₁-C₆)alkoxy groups, (C₁-C₆)alkyl-thio groups, halo(C₁-C₆)alkylthio groups, (C₂-C₆)alkenyl groups and (C₂-C₆)alkynyl groups], and R² is (iii) a group represented by the formula:
-N(R²¹)R²²
(wherein R²¹ is a hydrogen atom and R²² is selected from ② (C₁-C₁₂)alkyl groups, ③ unsubstituted halo(C₁-C₁₂)alkyl groups, ④ substituted halo(C₁-C₁₂)alkyl groups having a hydroxyl group or a (C₁-C₆)alkoxy group as the substituent, ⑤ (C₂-C₁₂)alkenyl groups, ⑥ (C₂-C₁₂)alkynyl groups, ⑦ (C₁-C₁₂)alkoxy(C₁-C₆)alkyl groups, ⑧ (C₁-C₁₂)alkylthio-(C₁-C₆)alkyl groups, ⑨ cyano(C₁-C₁₂)alkyl groups, ⑩ substituted cyano(C₁-C₆)alkyl groups having on the alkyl chain a substituent selected from the group consisting of (C₁-C₆)alkoxy groups, (C₂-C₆)alkenyloxy groups, (C₂-C₆)alkynyloxy groups, (C₁-C₆)alkylthio groups, (C₁-C₆)alkoxycarbonyl(C₁-C₆)alkoxy groups, (C₁-C₆)-alkoxycarbonyl(C₁-C₆)alkylthio groups, phenoxy group, phenylthio group and pyrazol-1-yl group, ⑬ hydroxy-(C₁-C₆)alkyl groups, ⑭ di (C₁-C₆)alkoxy(C₁-C₆)alkyl groups in which the (C₁-C₆)alkoxy groups may be the same or different, ⑮ unsubstituted (C₁-C₆)alkoxycarbonyl-(C₁-C₆)alkyl groups, ⑯ substituted (C₁-C₆)alkoxycarbonyl(C₁-C₆)alkyl groups having on the alkyl chain a substituent selected from the group consisting of (C₁-C₆)alkyl groups, (C₁-C₆)alkoxy groups, (C₂-C₆)-alkenyloxy groups, (C₂-C₆)alkynyloxy groups, (C₁-C₆)-alkylthio groups, (C₁-C₆)alkoxycarbonyl(C₁-C₆)alkoxy groups, (C₁-C₆)alkoxycarbonyl(C₁-C₆)alkylthio groups, phenyl group, phenyl(C₁-C₆)alkyl group, phenoxy group, phenylthio group and pyrazol-1-yl group, ⑰ unsubstituted phenyl groups, ⑱ substituted phenyl groups having one or more substituents which may be the same or different and are selected from the group consisting of ¹⁾ halogen atoms, ²⁾ nitro group, ³⁾ cyano group, ⁴⁾ (C₁-C₆)alkyl groups, ⁵⁾ halo(C₁₋C₆)alkyl groups, ⁶⁾ (C₁-C₆)alkoxy groups, ⁷⁾ halo(C₁-C₆)alkoxy groups, ⁸⁾ (C₁-C₆)alkylthio groups, ⁹⁾ halo(C₁-C₆)alkyl-thio groups, ¹⁰⁾ (C₂-C₆)alkenyl groups, ¹¹⁾ (C₂-C₆)-alkynyl groups, ¹²⁾ (C₁-C₆)alkylcarbonyl groups, ¹³⁾ carboxyl group, ¹⁴⁾ (C₁-C₁₂)alkoxycarbonyl groups, ¹⁵⁾ methylenedioxy group, ¹⁶⁾ phenyl group, ¹⁷⁾ groups represented by the formula: 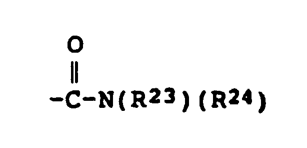 wherein R²³ and R²⁴, which may be the same or different, are hydrogen atoms; (C₁-C₁₂)alkyl groups; halo-(C₁-C₁₂)alkyl groups; (C₂-C₁₂)alkenyl groups ; (C₂-C₁₂)-alkynyl groups; unsubstituted phenyl groups ; substituted phenyl groups having one or more substituents which may be the same or different and are selected from the group consisting of halogen atoms, cyano group, nitro group, (C₁-C₆)alkyl groups and (C₁-C₆)alkoxy groups; or 5- or 6-membered heterocyclic rings containing, between adjacent carbon atoms of the carbon chain, one or more atoms which may be the same or different and are selected from the group consisting of oxygen atom, sulfur atom and nitrogen atom , 18) groups represented by the formula:
-SO₂-R²⁵
wherein R²⁵ is a hydrogen atom, hydroxyl group, a (C₁-C₁₂)alkyl group, a halo(C₁-C₆)alkyl group, an unsubstituted phenyl group, a substituted phenyl group having as the substituent(s) one or more substituents which may be the same or different and are selected from the group consisting of halogen atoms, (C₁-C₆)alkyl groups and (C₁-C₆)alkoxy groups, unsubstituted 1,2,3-thiadiazol-5-yl group, or substituted 1,2,3-thiadiazol-5-yl groups having halogen atom, (C₁-C₆)alkyl group or (C₁-C₆)alkoxy group as the substituent, 19) groups represented by the formula: 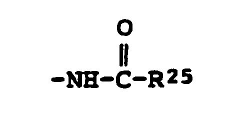 wherein R²⁵ is as defined above, ²⁰⁾ groups represented by the formula: 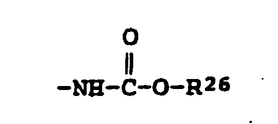 wherein R²⁶ is a (C₁-C₆)alkyl group, a phenyl group or a phenyl(C₁-C₆)alkyl group, ²¹⁾ groups represented by the formula:
-NH-SO₂-R²⁵
wherein R²⁵ is as defined above, ²²⁾ groups represented by the formula: 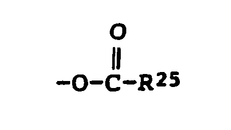 wherein R²⁵ is as defined above, and ²³⁾ groups represented by the formula: 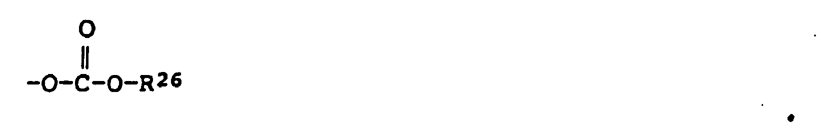 wherein R²⁶ is as defined above, ⑲ unsubstituted phenyl(C₁-C₆)alkyl groups, ⑳ substituted phenyl(C₁-C₆)-alkyl groups having on the ring one or more substituents which may be the same or different and are selected from the group consisting of halogen atoms, nitro group, cyano group, (C₁-C₆)alkyl groups, halo(C₁-C₆)alkyl groups, (C₁-C₆)alkoxy groups, halo(C₁-C₆)alkoxy groups, (C₁-C₆)alkylthio groups, halo(C₁-C₆)alkylthio groups, (C₂-C₆)alkenyl groups and (C₂-C₆)alkynyl groups, 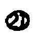 naphthyl groups, 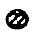 groups represented by the formula:
-(CH₂)ₚNHR¹⁸
wherein R¹⁸ is as defined above, and p is an integer of 1 to 12, or 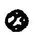 5- or 6-membered heterocyclic rings having one or more hetero atoms which may be the same or different and are selected from the group consisting of oxygen atom, sulfur atom and nitrogen atom, R²¹ and R²² being able to be taken together to represent 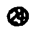 a (C₄-C₆)alkylene group which may contain an oxygen atom between adjacent carbon atoms of the carbon chain, or 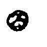 a diaminomethylene group for controlling agricultural and horticultural diseases which damage crops, fruit trees, vegetables, flowers and ornamental plants.

2. Use of an agricultural and horticultural disease controller according to claim 1, wherein R¹ is ① a hydrogen atom, ② a (C₁-C₁₂)alkyl group, ③ a halo-(C₁-C₁₂)alkyl group, ④ a (C₂-C₁₂)alkenyl group, ⑤ a halo(C₂-C₁₂)alkenyl group, ⑥ a (C₂-C₁₂)alkynyl group, ⑦ a halo(C₂-C₁₂)alkynyl group, ⑧ a (C₃-C₆)cycloalkyl group, ⑨ an unsubstituted phenyl group, or ⑩ a substituted phenyl group having one or more substituents which may be the same or different and are selected from the group consisting of halogen atoms, nitro group, cyano group, (C₁-C₆)alkyl groups, halo(C₁-C₆)alkyl groups, (C₁-C₆)alkoxy groups, halo(C₁-C₆)alkoxy groups, (C₁-C₆)alkylthio groups, halo(C₁-C₆)alkylthio groups, (C₂-C₆)alkenyl groups and (C₂-C₆)alkynyl groups, and R² is a group represented by the formula:
-N(R²¹)R²²
wherein:
R²¹ is a hydrogen atom and R²² is selected from ② (C₁-C₁₂) alkyl groups, ③ unsubstituted halo(C₁-C₁₂)alkyl groups, ⑤ (C₂-C₁₂)alkenyl groups, ⑥ (C₂-C₁₂)alkynyl groups, ⑦ (C₁-C₁₂)alkoxy(C₁-C₆)alkyl groups, ⑧ (C₁-C₁₂)alkylthio-(C₁-C₆)alkyl groups, ⑨ cyano(C₁-C₁₂)alkyl groups, ⑩ hydroxy-(C₁-C₆)alkyl groups, ⑮ unsubstituted (C₁-C₆)alkoxycarbonyl(C₁-C₆)alkyl groups, ⑰ unsubstituted phenyl groups, ⑱ substituted phenyl groups having one or more substituents which may be the same or different and are selected from the group consisting of ¹⁾ halogen atoms, ²⁾ nitro group, ³⁾ cyano group, ⁴⁾ (C₁-C₆)alkyl groups, ⁵⁾ halo(C₁-C₆)alkyl groups, ⁶⁾ (C₁-C₆)alkoxy groups, ⁷⁾ halo(C₁-C₆)alkoxy groups, ⁸⁾ (C₁-C₆)alkylthio groups, ¹²⁾ (C₁-C₆)alkylcarbonyl groups, ¹³⁾ carboxyl group, ¹⁴⁾ (C₁-C₁₂)alkoxycarbonyl groups, ¹⁵⁾ methylenedioxy group, ¹⁶⁾ phenyl group, ¹⁷⁾ groups represented by the formula: 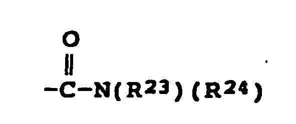
wherein R²³ and R²⁴, which may be the same or different, are hydrogen atoms; (C₁-C₁₂)alkyl groups; halo-(C₁-C₁₂)alkyl groups; (C₂-C₁₂)alkenyl groups; (C₂-C₁₂)-alkynyl groups; unsubstituted phenyl groups; substituted phenyl groups having one or more substituents which may be the same or different and are selected from the group consisting of halogen atoms, cyano group, nitro group, (C₁-C₆)alkyl groups and (C₁-C₆)alkoxy groups; or 5- or 6-membered heterocyclic rings containing, between adjacent carbon atoms of the carbon chain, one or more atoms which may be the same or different and are selected from the group consisting of oxygen atom, sulfur atom and nitrogen atom, ¹⁸⁾ groups represented by the formula:
-SO₂-R²⁵
wherein R²⁵ is a hydrogen atom, hydroxyl group, a (C₁-C₆)alkyl group, a halo(C₁-C₆)alkyl group, an unsubstituted phenyl group, a substituted phenyl group having as the substituent(s) one or more substituents which may be the same or different and are selected from the group consisting of halogen atoms, (C₁-C₆)alkyl groups and (C₁-C₆)alkoxy groups, unsubstituted 1,2,3-thiadiazol-5-yl group, or substituted 1,2,3-thiadiazol-5-yl groups having halogen atom, (C₁-C₆)alkyl group or (C₁-C₆)alkoxy group as the substituent, ¹⁹⁾ groups represented by the formula: 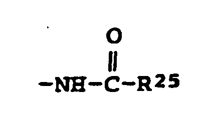 wherein R²⁵ is as defined above, ²⁰⁾ groups represented by the formula: 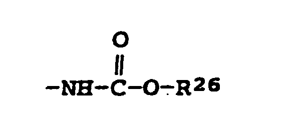 wherein R²⁶ is a (C₁-C₆)alkyl group, a phenyl group or a phenyl(C₁-C₆)alkyl group, ²¹⁾ groups represented by the formula:
-NH-SO₂-R²⁵
wherein R²⁵ is as defined above, ²²⁾ groups represented by the formula: 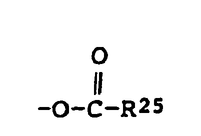 wherein R²⁵ is as defined above, and ²³⁾ groups represented by the formula: 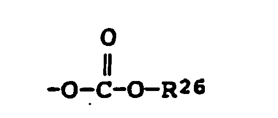 wherein R²⁶ is as defined above , ⑲ unsubstituted phenyl(C₁-C₆)alkyl groups, ⑳ substituted phenyl(C₁-C₆)-alkyl groups having on the ring one or more substituents which may be the same or different and are selected from the group consisting of halogen atoms, nitro group, cyano group, (C₁-C₆)alkyl groups, halo(C₁-C₆)alkyl groups, (C₁-C₆)alkoxy groups, and halo(C₁-C₆)alkoxy groups, 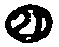 naphthyl groups, 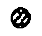 groups represented by the formula:
-(CH₂)ₚNHR¹⁸
wherein R¹⁸ is as defined above, and p is an integer of 1 to 12 , or 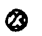 5- or 6-membered heterocyclic rings having one or more atoms which may be the same or different and are selected from the group consisting of oxygen atom, sulfur atom and nitrogen atom, R²¹ and R²² being able to be taken together to represent 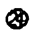 a (C₄-C₆)alkylene group which may contain an oxygen atom between adjacent carbon atoms of the carbon chain, or 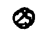 a diaminomethylene group for controlling agricultural and horticultural diseases which damage crops, fruit trees, vegetables, flowers and ornamental plants.

3. A method for controlling plant diseases which comprises applying an agricultural and horticultural disease controller set forth in any of claims 1 and 2 in a dosage of 0.1 g to 10 kg in terms of the active ingredient of the disease controller per 10 ares for protecting useful crops against the disease.

## Patentansprüche

1. Verwendung eines Bekämpfungsmittels für Krankheiten in der Landwirtschaft und im Gartenbau, umfassend als wirksamen Bestandteil ein 1,2,3-Thiadiazol-Derivat, dargestellt durch die allgemeine Formel (1), oder ein Salz davon 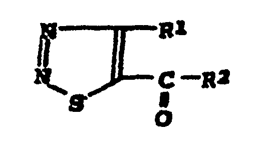 worin R¹ ist **(1)** ein Wasserstoffatom, **(2)** eine (C₁-C₁₂)Alkylgruppe, **(3)** eine Halogen-(C₁-C₁₂)alkylgruppe, **(4)** eine (C₂-C₁₂)Alkenylgruppe, **(5)** eine Halogen(C₂-C₁₂)-alkenylgruppe, **(6)** eine (C₂-C₁₂)Alkinylgruppe, **(7)** eine Halogen(C₂-C₁₂)alkinyl-gruppe, **(8)** eine (C₃-C₆)Cycloalkylgruppe, **(9)** eine unsubstituierte Phenylgruppe, **(10)** eine substituierte Phenylgruppe mit einem oder mehreren Substituenten, welche gleich oder verschieden sein können und welche ausgewählt werden aus der Gruppe bestehend aus Halogenatomen, Nitrogruppe, Cyanogruppe, (C₁-C₆)Alkylgruppen, Halogen(C₁-C₆)alkylgruppen, (C₁-C₆)Alkoxygruppen, Halogen(C₁-C₆)alkoxygruppen, (C₁-C₆)Alkylthiogruppen, Halogen(C₁-C₆)alkylthiogruppen, (C₂-C₆)Alkenylgruppen und (C₂-C₆)Alkinylgruppen, **(11)** eine Gruppe, dargestellt durch die Formel: 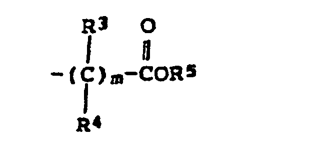 worin R³ und R⁴, welche gleich oder verschieden sein können, Wasserstoffatome, (C₁-C₁₂)Alkylgruppen oder Halogen(C₁-C₁₂)alkylgruppen sind, m Null oder eine ganze Zahl von 1 bis 6 ist, und R⁵ ist ein Wasserstoffatom; eine (C₁-C₁₂)Alkylgruppe; eine Halogen(C₁-C₁₂)alkylgruppe; eine (C₂-C₁₂)Alkenylgruppe; eine (C₂-C₁₂)Alkenylgruppe; eine unsubstituierte Phenylgruppe; eine substituierte Phenylgruppe mit einem oder mehreren Substituenten, welche gleich oder verschieden sein können und ausgewählt werden aus der Gruppe bestehend aus Halogenatomen, Nitrogruppe, Cyanogruppe, (C₁-C₆)Alkylgruppen; Halogen(C₁-C₆)alkylgruppen; (C₁-C₆)Alkoxygruppen, Halogen(C₁-C₆)alkoxygruppen, (C₁-C₆)Alkylthiogruppen, Halogen(C₁-C₆) alkylthiogruppen, (C₂-C₆)-Alkenylgruppen und (C₂-C₆)Alkinylgruppen; eine unsubstituierte Phenyl(C₁-C₆)alkylgruppe; oder eine substituierte Phenyl(C₁-C₆)alkylgruppe mit einem oder mehreren Substituenten am Ring, welche gleich oder verschieden sein können und ausgewählt werden aus der Gruppe bestehend aus Halogenatomen, Nitrogruppe, Cyanogruppe, (C₁-C₆)Alkylgruppen, Halogen(C₁-C₆)alkylgruppen, (C₁-C₆)Alkoxygruppen, Halogen(C₁-C₆)alkoxygruppen, (C₁-C₆)Alkylthiogruppen, Halogen(C₁-C₆)alkylthiogruppen, (C₂-C₆)Alkenylgruppen und (C₂-C₆)Alkinylgruppen, **(12)** eine Gruppe, dargestellt durch die Formel: 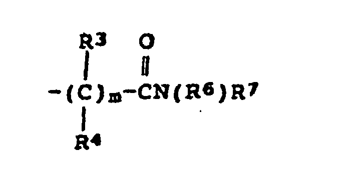 worin R³, R⁴ und m wie oben definiert sind und R⁶ und R⁷, welche gleich oder verschieden sein können, sind Wasserstoffatome; (C₁-C₁₂)Alkylgruppen; Halogen(C₁-C₁₂)alkylgruppen; (C₂-C₁₂)Alkenylgruppen; (C₂-C₁₂)Alkinylgruppen; (C₁-C₁₂)Alkoxy-(C₁-C₆)alkylgruppen; (C₁-C₁₂)Alkylthio(C₁-C₆)alkylgruppen; Cyano(C₁-C₁₂)alkylgruppen; unsubstituierte Phenylgruppen; substituierte Phenylgruppen mit einem oder mehreren Substituenten, welche gleich oder verschieden sein können und ausgewählt werden aus der Gruppe bestehend aus Halogenatomen, Nitrogruppe, Cyanogruppe, (C₁-C₆)Alkylgruppen, Halogen(C₁-C₆)alkylgruppen, (C₁-C₆)Alkoxy-gruppen, Halogen(C₁-C₆)alkoxygruppen, (C₁-C₆)Alkylthiogruppen, Halogen(C₁-C₆)alkylthiogruppen, (C₂-C₆)Alkenylgruppen und (C₂-C₆)Alkinylgruppen; unsubstituierte Phenyl(C₁-C₆)alkylgruppen; oder substituierte Phenyl(C₁-C₆)alkylgruppen mit einem oder mehreren Substituenten am Ring, welche gleich oder verschieden sein können und ausgewählt werden aus der Gruppe bestehend aus Halogenatom, Nitrogruppe, Cyanogruppe, (C₁-C₆)Alkylgruppen, Halogen(C₁-C₆)alkylgruppen, (C₁-C₆)-Alkoxygruppen, Halogen(C₁-C₆)alkoxygruppen, (C₁-C₆)Alkylthiogruppen, Halogen(C₁-C₆)alkylthiogruppen, (C₂-C₆)Alkenylgruppen und (C₂-C₆)Alkinylgruppen; R⁶ und R⁷ miteinander verbunden sein können, um eine (C₄-C₆)Alkylengruppe darzustellen, welche ein Sauerstoffatom zwischen nebeneinander liegenden Kohlenstoffatomen der Kohlenstoffkette enthalten kann, **(13)** eine Gruppe dargestellt durch die Formel: 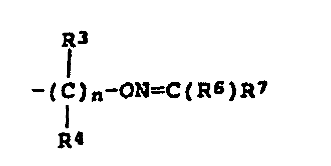 worin R³, R⁴, R⁶ und R⁷ wie oben definiert sind und n eine ganze Zahl von 1 bis 6 ist, oder **(14)** eine Gruppe, dargestellt durch die Formel: 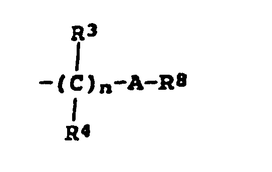 worin R³, R⁴ und n wie oben definiert sind, A ist
-O- , 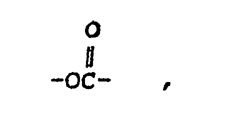 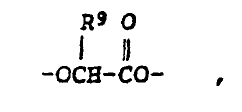 worin R⁹ ein Wasserstoffatom, eine (C₁-C₁₂)Alkylgruppe oder eine Halogen(C₁-C₁₂)alkylgruppe ist,
-S- ,
-SO- ,
-SO₂-, 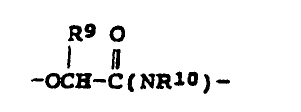 worin R⁹ wie oben definiert ist, und R¹⁰ ein Wasserstoffatom, eine (C₁-C₁₂)Alkylgruppe oder eine Halogen(C₁-C₁₂)alkylgruppe ist, R⁹ und R¹⁰ miteinander verbunden sein können, um eine (C₄-C₆)Alkylengruppe darzustellen, welche zwischen benachbarten Kohlenstoffatomen der Kohlenstoffkette ein Sauerstoffatom, ein Schwefelatom oder
N-R¹¹
enthalten kann, worin R¹¹ ein Wasserstoffatom, eine (C₁-C₁₂)Alkylgruppe oder eine Halogen(C₁-C₁₂)alkylgruppe ist, oder
-N(R¹⁰)-
worin R¹⁰ wie oben definiert ist und R⁸ ist ein Wasserstoffatom; eine (C₁-C₁₂)Alkylgruppe; eine Halogen(C₁-C₁₂)Alkylgruppe; eine (C₂-C₁₂)Alkenylgruppe; eine (C₂-C₁₂)Alkinylgruppe; eine (C₁-C₁₂)Alkoxy(C₁-C₁₂)alkylgruppe; eine (C₁-C₁₂)Alkylthio(C₁-C₁₂)alkylgruppe; eine Cyano(C₁-C₁₂)alkylgruppe; eine unsubstituierte Phenylgruppe; eine substituierte Phenylgruppe mit einem oder mehreren Substituenten, die gleich oder verschieden sein können und ausgewählt werden aus der Gruppe bestehend aus Halogenatomen, Nitrogruppe, Cyanogruppe, (C₁-C₆)Alkylgruppen, Halogen(C₁-C₆)alkylgruppen, (C₁-C₆)Alkoxygruppen, Halogen(C₁-C₆)alkoxygruppen, (C₁-C₆)Alkylthiogruppen, Halogen(C₁-C₆)alkylthiogruppen, (C₂-C₆)Alkenylgruppen und (C₂-C₆)-Alkinylgruppen; eine unsubstituierte Phenyl(C₁-C₆)alkylgruppe; eine substituierte Phenyl(C₁-C₆)alkylgruppe mit einem oder mehreren Substituenten am Ring, welche gleich oder verschieden sein können und ausgewählt werden aus der Gruppe bestehend aus Halogenatomen, Nitrogruppe, Cyanogruppe, (C₁-C₆)Alkylgruppen, Halogen(C₁-C₆)alkylgruppen, (C₁-C₆)Alkoxygruppen, Halogen(C₁-C₆)alkoxygruppen, (C₁-C₆)Alkylthiogruppen, Halogen(C₁-C₆)alkylthiogruppen, (C₂-C₆)Alkenylgruppen und (C₂-C₆)Alkinylgruppen; ein unsubstituierter 5- oder 6-gliedriger heterocyclischer Ring mit einem oder mehreren Heteroatomen, welche gleich oder verschieden sein können und ausgewählt werden aus der Gruppe bestehend aus Sauerstoffatom, Schwefelatom und Stickstoffatom; oder ein substituierter 5- oder 6-gliedriger heterocyclischen Ring mit einem oder mehreren Heteroatomen, welche gleich oder verschieden sein können und ausgewählt werden aus der Gruppe bestehend aus Sauerstoffatom, Schwefelatom und Stickstoffatom, wobei der substituierte heterocyclische Ring einen oder mehrere Substituenten aufweist, welche gleich oder verschieden sein können und ausgewählt werden aus der Gruppe bestehend aus Halogenatomen, Nitrogruppe, Cyanogruppe, (C₁-C₆)Alkylgruppen, Halogen(C₁-C₆)alkylgruppen, (C₁-C₆)Alkoxygruppen, Halogen(C₁-C₆)alkoxygruppen, (C₁-C₆)Alkylthiogruppen, Halogen(C₁-C₆)-alkylthiogruppen, (C₂-C₆)Alkenylgruppen und (C₂-C₆)Alkinylgruppen aufweist; und R² ist eine Gruppe, dargestellt durch die Formel:
-N(R²¹)R²²
worin R²¹ ein Wasserstoffatom ist und R²² ausgewählt ist aus **(2)** (C₁-C₁₂)Alkylgruppen, **(3)** unsubstituierte Halogen(C₁-C₁₂)Alkylgruppen, **(4)** substituierte Halogen(C₁-C₁₂)Alkylgruppen mit einer Hydroxylgruppe oder einer (C₁-C₆)Alkoxygruppe als den Substituenten, **(5)** (C₂-C₁₂)Alkenylgruppen, **(6)** (C₂-C₁₂)Alkinylgruppen, **(7)** (C₁-C₁₂)Alkoxy(C₁-C₆)alkylgruppen, **(8)** (C₁₋₁₂)Alkylthio(C₁-C₆)alkylgruppen, **(9)** Cyano(C₁-C₁₂)alkylgruppen, **(10)** substituierte Cyano(C₁-C₆)alkylgruppen mit einem Substituenten an der Alkylkette ausgewählt aus der Gruppe bestehend aus (C₁-C₆)Alkoxygruppen, (C₂-C₆)Alkenyloxygruppen, (C₂-C₆)Alkinyloxygruppen, (C₁-C₆)Alkylthiogruppen, (C₁-C₆)Alkoxycarbonyl(C₁-C₆)alkoxygruppen, (C₁-C₆)Alkoxycarbonyl(C₁-C₆)alkylthiogruppen, Phenoxygruppe, Phenylthiogruppe und Pyrazol-1-yl-Gruppe, **(13)** Hydroxy(C₁-C₆)alkylgruppen, **(14)** Di(C₁-C₆)alkoxy(C₁-C₆)alkylgruppen, in welchen die (C₁-C₆)Alkoxygruppen gleich oder verschieden sein können, **(15)** unsubstituierte (C₁-C₆)Alkoxycarbonyl(C₁-C₆)alkylgruppen, **(16)** substituierte (C₁-C₆)Alkoxycarbonyl(C₁-C₆)alkylgruppen mit einem Substituenten an der Alkylkette ausgewählt aus der Gruppe bestehend aus (C₁-C₆)Alkylgruppen, (C₁-C₆)Alkoxygruppen, (C₂-C₆)Alkenyloxygruppen, (C₂-C₆)Aikinyioxygruppen, (C₁-C₆)Alkylthiogruppen, (C₁-C₆)Alkoxycarbonyl(C₁-C₆)alkoxygruppen, (C₁-C₆)Alkoxycarbonyl(C₁-C₆)alkylthiogruppen, Phenylgruppe, Phenyl(C₁-C₆)alkylgruppe, Phenoxygruppe, Phenylthiogruppe und Pyrazol-1-ylgruppe, **(17)** unsubstituierte Phenylgruppen, **(18)** substituierte Phenylgruppen mit einem oder mehreren Substituenten, welche gleich oder verschieden sein können und ausgewählt werden aus der Gruppe bestehend aus 1 ) Halogenatomen, 2) Nitrogruppe, 3) Cyanogruppe, 4) (C₁-C₆)Alkylgruppen, 5) Halogen (C₁-C₆)alkylgruppen, 6) (C₁-C₆)Alkoxygruppen, 7) Halogen(C₁-C₆)alkoxygruppen, 8) (C₁-C₆)Alkylthiogruppen, 9) Halogen(C₁-C₆)alkylthiogruppen, 10) (C₂-C₆)Alkenylgruppen, 11) (C₂-C₆)Alkinylgruppen, 12) (C₁-C₆)Alkylcarbonylgruppen, 13) Carboxylgruppen, 14) (C₁-C₁₂)Alkoxycarbonylgruppen, 15) Methylendioxygruppe, 16) Phenylgruppe, 17) Gruppen dargestellt durch die Formel: 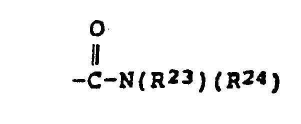 worin R²³ und R²⁴, welche gleich oder verschieden sein können, sind Wasserstoffatome; (C₁-C₁₂)Alkylgruppen; Halogen(C₁-C₁₂)alkylgruppen; (C₂-C₁₂)Alkenylgruppen, (C₂-C₁₂)Alkinylgruppen; unsubstituierte Phenylgruppen; substituierte Phenylgruppen mit einem oder mehreren Substituenten, welche gleich oder verschieden sein können und ausgewählt werden aus der Gruppe bestehend aus Halogenatomen, Cyanogruppe, Nitrogruppe, (C₁-C₆)Alkylgruppen und (C₁-C₆)Alkoxygruppen; oder 5- oder 6-gliedrige heterocyclische Ringe enthaltend zwischen benachbarten Kohlenstoffatomen der Kohlenstoffkette, ein oder mehrere Atome, welche gleich oder verschieden sein können und ausgewählt werden aus der Gruppe bestehend aus Sauerstoffatom, Schwefelatom und Stickstoffatom, 18) Gruppen dargestellt durch die Formel:
-SO₂-R²⁵
worin R²⁵ ein Wasserstoffatom ist, Hydroxylgruppe, eine (C₁-C₁₂)Alkylgruppe, eine Halogen(C₁-C₆)alkylgruppe, eine unsubstituierte Phenylgruppe, eine substituierte Phenylgruppe mit einem oder mehreren Substituenten, welche gleich oder verschieden sein können und ausgewählt werden aus der Gruppe bestehend aus Halogenatomen, (C₁-C₆)Alkylgruppen und (C₁-C₆)Alkoxygruppen, unsubstituierte 1,2,3-Thiadiazol-5-yl-Gruppe oder substituierte 1,2,3-Thiadiazol-5-yl-Gruppen mit einem
Halogenatom, (C₁-C₆)Alkylgruppe oder (C₁-C₆)Alkoxygruppe als den Substituenten, 19) Gruppen dargestellt durch die Formel: 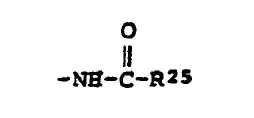 worin R²⁵ wie oben definiert ist, 20) Gruppen dargestellt durch die Formel: 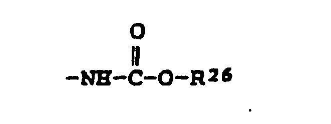 worin R²⁶ eine (C₁-C₆)Alkylgruppe, eine Phenylgruppe oder eine Phenyl(C₁-C₆)-alkylgruppe ist, 21) Gruppen dargestellt durch die Formel:
-NH-SO₂-R²⁵
worin R²⁵ wie oben definiert ist, 22) Gruppen dargestellt durch die Formel: 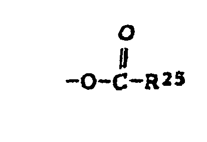 worin R²⁵ wie oben definiert ist und 23) Gruppen dargestellt durch die Formel: 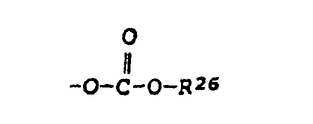 worin R²⁶ wie oben definiert ist, **(19)** unsubstituierte Phenyl(C₁-C₆)alkylgruppen, **(20)** substituierte Phenyl(C₁-C₆)alkylgruppen mit einem oder mehreren Substituenten am Ring, welche gleich oder verschieden sein können und ausgewählt werden aus der Gruppe bestehend aus Halogenatomen, Nitrogruppe, Cyanogruppe, (C₁-C₆)Alkylgruppen, Halogen(C₁-C₆)alkylgruppen, (C₁-C₆)Alkoxygruppen, Halogen(C₁-C₆)-alkoxygruppen, (C₁-C₆)Alkylthiogruppen, Halogen(C₁-C₆alkylthiogruppen, (C₂-C₆)-Alkenylgruppen und (C₂-C₆)Alkinylgruppen, **(21)** Naphthylgruppen, **(22)** Gruppen dargestellt durch die Formel:
-(CH₂)ₚNHR¹⁸
worin R¹⁸ wie oben definiert ist, und p eine ganze Zahl von 1 bis 12 ist, oder **(23)** 5- oder 6-gliedrige heterocyclische Ringe mit einem oder mehreren Heteroatomen, welche gleich oder verschieden sein können und ausgewählt werden aus der Gruppe bestehend aus Sauerstoffatom, Schwefelatom und Stickstoffatom, R²¹ und R²² miteinander verbunden sein können, um **(24)** eine (C₄-C₆)Alkylengruppe darzustellen, welche ein Sauerstoffatom zwischen den benachbarten Kohlenstoffatomen der Kohlenstoffkette enthalten kann oder **(25)** eine Diaminomethylengruppe
zur Bekämpfung von Krankheiten in der Landwirtschaft und im Gartenbau, welche Feldfrüchte, Obstbäume, Gemüse, Blumen und Zierpflanzen beschädigen.

2. Verwendung eines Bekämpfungsmittels für Krankheiten in der Landwirtschaft und im Gartenbau gemäß Anspruch 1, worin R¹ ist **(1)** ein Wasserstoffatom, **(2)** eine (C₁-C₁₂)Alkylgruppe, **(3)** eine Halogen(C₁-C₁₂)alkylgruppe, **(4)** eine (C₂-C₁₂)Alkenylgruppe, **(5)** eine Halogen(C₂-C₁₂)alkenylgruppe, **(6)** eine (C₂-C₁₂)Alkinylgruppe, **(7)** eine Halogen(C₂-C₁₂)alkinylgruppe, **(8)** eine (C₃-C₆)Cycloalkylgruppe, **(9)** eine unsubstituierte Phenylgruppe, oder **(10)** eine substituierte Phenylgruppe mit einem oder mehreren Substituenten, welche gleich oder verschieden sein können und ausgewählt werden aus der Gruppe bestehend aus Halogenatomen, Nitrogruppe, Cyanogruppe, (C₁-C₆)Alkylgruppen, Halogen(C₁-C₆)alkylgruppen, (C₁-C₆)Alkoxygruppen, Halogen(C₁-C₆)alkoxygruppen, (C₁-C₆)Alkylthiogruppen, Halogen(C₁-C₆)alkylthiogruppen, (C₂-C₆)Alkenylgruppen und (C₂-C₆)Alkinylgruppen, und R² ist eine Gruppe dargestellt durch die Formel:
-N(R²¹)R²²
worin R²¹ ein Wasserstoffatom ist und R²² ausgewählt ist aus **(2)** (C₁-C₁₂)Alkylgruppen, **(3)** unsubstituierte Halogen(C₁-C₁₂)alkylgruppen, **(5)** (C₂-C₁₂)Alkenylgruppen, **(6)** (C₂-C₁₂)Alkinylgruppen, **(7)** (C₁-C₁₂)Alkoxy(C₁-C₆)alkylgruppen, **(8)** (C₁-C₁₂)Alky/thio(C₁-C₆)alkylgruppen, **(9)** Cyano(C₁-C₁₂)alkylgruppen, **(11)** unsubstituierte Carbamoyl(C₁-C₆)alkylgruppen, **(13)** Hydroxy(C₁-C₆)alkylgruppen, **(15)** unsubstituierte (C₁-C₆)Alkoxycarbonyl(C₁-C₆)alkylgruppen, **(17)** unsubstituierte Phenylgruppen, mehreren Substituenten, welche gleich oder verschieden sein können und ausgewählt werden aus der Gruppe bestehend aus 1) Halogenatomen, 2) Nitrogruppe, 3) Cyanogruppe, 4) (C₁-C₆)Alkylgruppen; 5) Halogen(C₁-C₆)alkylgruppen, 6) (C₁-C₆) Alkoxygruppen, 7) Halogen(C₁-C₆)alkoxygruppen, 8) (C₁-C₆)Alkylthiogruppen, 12) (C₁-C₆)Alkylcarbonyl-gruppen, 13) Carboxylgruppen, 14) (C₁-C₁₂)Alkoxycarbonylgruppen, 15) Methylendioxygruppe, 16) Phenylgruppe, 17) Gruppen dargestellt durch die Formel: 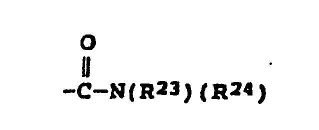 worin R²³ und R²⁴, welche gleich oder verschieden sein können, sind Wasserstoffatome; (C₁-C₁₂)Alkylgruppen ; Halogen(C₁-C₁₂)alkylgruppen ; (C₂-C₁₂)Alkenylgruppen, (C₂-C₁₂)Alkinylgruppen ; unsubstituierte Phenylgruppen; substituierte Phenylgruppen mit einem oder mehreren Substituenten, welche gleich oder verschieden sein können und ausgewählt werden aus der Gruppe bestehend aus Halogenatomen, Cyanogruppe, Nitrogruppe, (C₁-C₆)Alkylgruppen und (C₁-C₆)Alkoxygruppen ; oder 5- oder 6-gliedrige heterocyclische Ringen enthaltend zwischen benachbarten Kohlenstoffatomen der Kohlenstoffkette ein oder mehrere Atome, welche gleich oder verschieden sein können und ausgewählt werden aus der Gruppe bestehend aus Sauerstoffatom, Schwefelatom und Stickstoffatom, 18) Gruppen dargestellt durch die Formel:
-SO₂-R²⁵
worin R²⁵ ist ein Wasserstoffatom, Hydroxylgruppe, eine (C₁-C₁₂)Alkylgruppe, eine Halogen(C₁-C₆)alkylgruppe, eine unsubstituierte Phenylgruppe, eine substituierte Phenylgruppe mit einem oder mehreren Substituenten, welche gleich oder verschieden sein können und ausgewählt werden aus der Gruppe bestehend aus Halogenatomen, (C₁-C₆)Alkylgruppen und (C₁-C₆)Alkoxygruppen als Substituenten, unsubstituierte 1,2,3-Thiadiazol-5-yl-Gruppe oder substituierte 1,2,3-Thiadiazol-5-yl-Gruppen mit einem Halogenatom, (C₁-C₆)Alkylgruppe oder (C₁-C₆)Alkoxygruppe als den Substituenten, 19) Gruppen dargestellt durch die Formel: 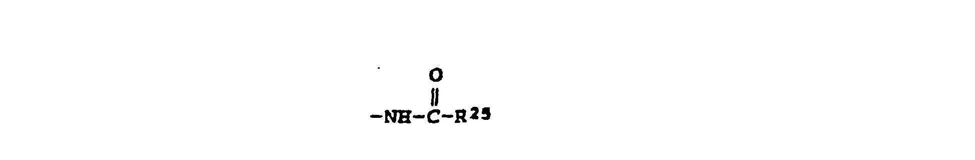 worin R²⁵ wie oben definiert ist, 20) Gruppen dargestellt durch die Formel: 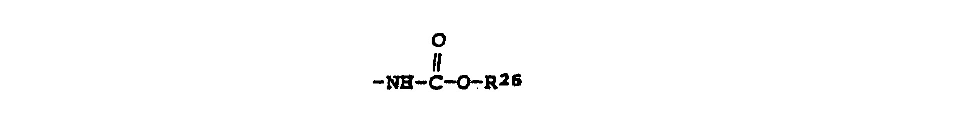 worin R²⁶ eine (C₁-C₆)Alkylgruppe, eine Phenylgruppe oder Phenyl(C₁-C₆)alkylgruppe ist, 21) Gruppen dargestellt durch die Formel:
-NH-SO₂-R²⁵
worin R²⁵ wie oben definiert ist, 22) Gruppen dargestellt durch die Formel: 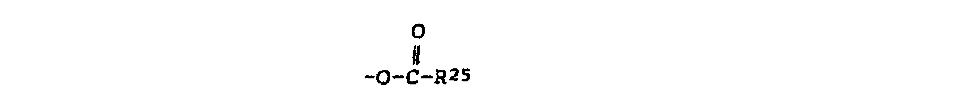 worin R²⁵ wie oben definiert ist, und 23) Gruppen dargestellt durch die Formel: 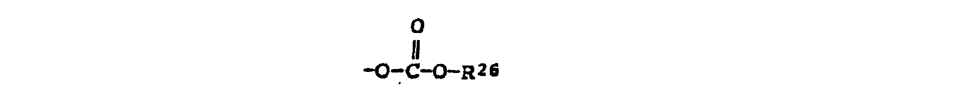 worin R²⁶ wie oben definiert ist, **(19)** unsubstituierte Phenyl(C₁-C₆)alkylgruppen, **(20)** substituierte Phenyl(C₁-C₆)alkylgruppen mit einem oder mehreren Substituenten am Ring, welche gleich oder verschieden sein können und ausgewählt werden aus der Gruppe bestehend aus Halogenatomen, Nitrogruppe, Cyanogruppe, (C₁-C₆)Alkylgruppen, Halogen(C₁-C₆)alkylgruppen, (C₁-C₆)Alkoxygruppen und Halogen(C₁-C₆)alkoxygruppen, **(21)** Naphthylgruppen, **(22)** Gruppen dargestellt durch die Formel:
-(CH₂)ₚNHR¹⁸
worin R¹⁸ wie oben definiert ist, und p eine ganze Zahl von 1 bis 12 ist, oder **(23)** 5- oder 6-gliedrige heterocyclische Ringe mit einem oder mehreren Heteroatomen, welche gleich oder verschieden sein können und ausgewählt werden aus der Gruppe bestehend aus Sauerstoffatom, Schwefelatom und Stickstoffatom, R²¹ und R²² miteinander verbunden sein können, um **(24)** eine (C₄-C₆)Alkylengruppe darzustellen, welche ein Sauerstoffatom zwischen den benachbarten Kohlenstoffatomen der Kohlenstoffkette enthalten kann, oder **(25)** eine Diaminomethylengruppe zur Bekämpfung von Krankheiten in der Landwirtschaft und im Gartenbau, welche Feldfrüchte, Obstbäume, Gemüse, Blumen und Zierpflanzen beschädigen.

3. Ein Verfahren zur Bekämpfung von Pflanzenkrankheiten, welches das Aufbringen eines Bekämpfungsmittels für Krankheiten in der Landwirtschaft und im Gartenbau wie in den Ansprüchen 1 und 2 beschrieben, in einer Dosierung von 0,1 g bis 10 kg bezogen auf den Wirkstoff des Krankheitsbekämpfungsmittels pro 10 Ar umfasst zum Schutz von nutzbaren Feldfrüchten gegen die Krankheit.

## Revendications

1. Utilisation d'un agent de lutte contre les maladies dans les domaines agricole et horticole comprenant en tant qu'ingrédient actif un dérivé de 1,2,3-thiadiazole représenté par la formule générale (I), ou un sel de celui-ci 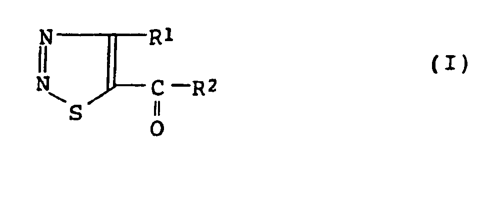 dans laquelle R¹ représente (1) un atome d'hydrogène, (2) un groupe alkyle en C₁-C₁₂, (3) un groupe halogéno (alkyle en C₁-C₁₂), (4) un groupe alcényle en C₂-C₁₂, (5) un groupe halogéno(alcényle en C₂-C₁₂), (6) un groupe alcynyle en C₂-C₁₂, (7) un groupe halogéno(alcynyle en C₂-C₁₂), (8) un groupe cycloalkyle en C₃-C₆, (9) un groupe phényle non substitué, (10) un groupe phényle substitué ayant un ou plusieurs substituants qui peuvent être identiques ou différents et sont choisis dans le groupe constitué par les atomes d'halogène, un groupe nitro, un groupe cyano, les groupes alkyle en C₁-C₆, les groupes halogéno (alkyle en C₁-C₆), les groupes alcoxy en C₁-C₆, les groupes halogéno (alcoxy en C₁-C₆), les groupes (alkyle en C₁-C₆) thio, les groupes halogéno(alkyle en C₁-C₆) thio, les groupes alcényle en C₂-C₆, et les groupes alcynyle en C₂-C₆, (11) un groupe représenté par la formule : 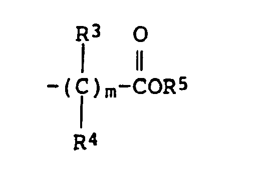 dans laquelle R³ et R⁴, qui peuvent être identiques ou différents, représentent des atomes d'hydrogène, des groupes alkyle en C₁-C₁₂ ou des groupes halogéno(alkyle en C₁-C₁₂), m vaut zéro ou est un nombre entier de 1 à 6, et R⁵ représente un atome d'hydrogène; un groupe alkyle en C₁-C₁₂; un groupe halogéno(alkyle en C₁-C₁₂) ; un groupe alcényle en C₂-C₁₂ ; un groupe alcynyle en C₂-C₁₂ ; un groupe phényle non substitué; un groupe phényle substitué ayant un ou plusieurs substituants qui peuvent être identiques ou différents et sont choisis dans le groupe constitué par les atomes d'halogène, un groupe nitro, un groupe cyano, les groupes alkyle en C₁-C₆, les groupes halogéno(alkyle en C₁-C₆), les groupes alcoxy en C₁-C₆, les groupes halogéno (alcoxy en C₁-C₆) , les groupes (alkyle en C₁-C₆) thio, les groupes halogéno(alkyle en C₁-C₆) thio, les groupes alcényle en C₂-C₆ et les groupes alcynyle en C₂-C₆; un groupe phényl(alkyle en C₁-C₆) non-substitué; ou un groupe phényl(alkyle en C₁-C₆) substitué ayant sur le noyau un ou plusieurs substituants qui peuvent être identiques ou différents et sont choisis dans le groupe constitué par les atomes d'halogène, un groupe nitro, un groupe cyano, les groupes alkyle en C₁-C₆, les groupes halogéno(alkyle en C₁-C₆), les groupes alcoxy en C₁-C₆, les groupes halogéno (alcoxy en C₁-C₆), les groupes (alkyle en C₁-C₆) thio, les groupes halogéno(alkyle en C₁-C₆) thio, les groupes alcényle en C₂-C₆ et les groupes alcynyle en C₂-C₆), (12) un groupe représenté par la formule : 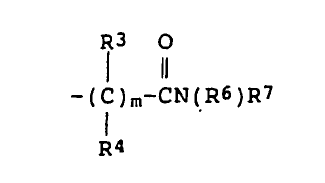 dans laquelle R³, R⁴ et m sont comme définis ci-dessus, et R⁶ et R⁷, qui peuvent être identiques ou différents, représentent des atomes d'hydrogène ; des groupes alkyle en C₁-C₁₂ ; des groupes halogéno (alkyle en C₁-C₁₂) ; des groupes alcényle en C₂-C₁₂; des groupes alcynyle en C₂-C₁₂ ; des groupes (alcoxy en C₁-C₁₂) (alkyle en C₁-C₆) ; des groupes (alkyle en C₁-C₁₂) thio-(alkyle en C₁-C₆) ; des groupes cyano(alkyle en C₁-C₁₂) ; des groupes phényle non-substitués ; des groupes phényle substitués ayant un ou plusieurs substituants qui peuvent être identiques ou différents et sont choisis dans le groupe constitué par les atomes d'halogène, un groupe nitro, un groupe cyano, les groupes alkyle en C₁-C₆, les groupes halogéno(alkyle en C₁-C₆), les groupes alcoxy en C₁-C₆, les groupes halogéno (alcoxy en C₁-C₆), les groupes (alkyle en C₁-C₆) thio, les groupes halogéno(alkyle en C₁-C₆) thio, les groupes alcényle en C₂-C₆ et les groupes alcynyle en C₂-C₆; des groupes phényl (alkyle en C₁-C₆) non-substitués; ou des groupes phényl(alkyle en C₁-C₆) substitués ayant sur le noyau un ou plusieurs substituants qui peuvent être identiques ou différents et sont choisis dans le groupe constitué par les atomes d'halogène, un groupe nitro, un groupe cyano, les groupes alkyle en C₁-C₆, les groupes halogéno(alkyle en C₁-C₆) , les groupes alcoxy en C₁-C₆, les groupes halogéno (alcoxy en C₁-C₆), les groupes (alkyle en C₁-C₆) thio, les groupes halogéno(alkyle en C₁-C₆) thio, les groupes alcényle en C₂-C₆ et les groupes alcynyle en C₂-C₆ ; R⁶ et R⁷ pouvant être pris conjointement afin de représenter un groupe alkylène en C₄-C₆ qui peut contenir un atome d'oxygène entre des atomes de carbone adjacents de la chaîne carbonée, (13) un groupe représenté par la formule: 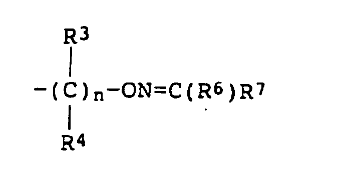 dans laquelle R³, R⁴, R⁶ et R⁷ sont comme définis ci-dessus, et n est un nombre entier de 1 à 6, ou (14) un groupe représenté par la formule : 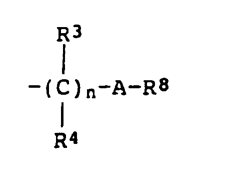 dans laquelle R³, R⁴ et n sont comme définis ci-dessus, A est
-O-, 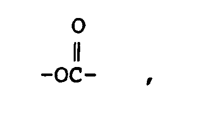 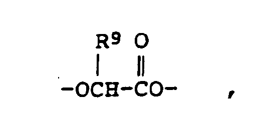 dans lequel R⁹ représente un atome d'hydrogène, un groupe alkyle en C₁-C₁₂, un groupe halogéno(alkyle en C₁-C₁₂),
-S- ,
-SO- ,
-SO₂- , 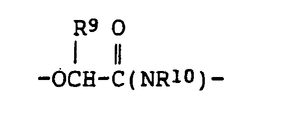 dans lequel R⁹ est comme défini ci-dessus, et R¹⁰ représente un atome d'hydrogène, un groupe alkyle en C₁-C₁₂, ou un groupe halogéno(alkyle en C₁-C₁₂), R⁹ et R¹⁰ pouvant être pris conjointement afin de représenter un groupe alkylène en C₄-C₆ qui peut contenir, entre des atomes de carbone adjacents de la chaîne carbonée, un atome d'oxygène, un atome de soufre ou
N-R¹¹
dans lequel R¹¹ représente un atome d'hydrogène, un groupe alkyle en C₁-C₁₂ ou un groupe halogéno(alkyle en C₁-C₁₂), ou
-N(R¹⁰)-
dans lequel R¹⁰ est comme défini ci-dessus, et R⁸ représente un atome d'hydrogène ; un groupe alkyle en C₁-C₁₂ ; un groupe halogéno (alkyle en C₁-C₁₂); un groupe alcényle en C₂-C₁₂; un groupe alcynyle en C₂-C₁₂ ; un groupe (alcoxy en C₁-C₁₂) (alkyle en C₁-C₁₂) ; un groupe (alkyle en C₁-C₁₂) thio-(alkyle en C₁-C₁₂); un groupe cyano (alkyle en C₁-C₁₂); un groupe phényle non substitué ; un groupe phényle substitué ayant un ou plusieurs substituants qui peuvent être identiques ou différents et sont choisis dans le groupe constitué par les atomes d'halogène, un groupe nitro, un groupe cyano, les groupes alkyle en C₁-C₆, les groupes halogéno (alkyle en C₁-C₆), les groupes alcoxy en C₁-C₆, les groupes halogéno (alcoxy en C₁-C₆), les groupes (alkyle en C₁-C₆) thio, les groupes halogéno(alkyle en C₁-C₆) thio, les groupes alcényle en C₂-C₆ et les groupes alcynyle en C₂-C₆ ; un groupe phényl(alkyle en C₁-C₆) non substitué ; un groupe phényl (alkyle en C₁-C₆) substitué ayant sur le noyau un ou plusieurs substituants pouvant être identiques ou différents et sont choisis dans le groupe constitué par les atomes d'halogène, un groupe nitro, un groupe cyano, les groupes alkyle en C₁-C₆, les groupes halogéno(alkyle en C₁-C₆), les groupes alcoxy en C₁-C₆, les groupes halogéno(alcoxy en C₁-C₆), les groupes (alkyle en C₁-C₆) thio, les groupes halogéno(alkyle en C₁-C₆) thio, les groupes alcényle en C₂-C₆ et les groupes alcynyle en C₂-C₆; un noyau hétérocyclique non-substitué à 5 ou 6 chaînons ayant un ou plusieurs hétéroatomes qui peuvent être identiques ou différents et sont choisis dans le groupe constitué par un atome d'oxygène, un atome de soufre et un atome d'azote ; ou un noyau hétérocyclique substitué à 5 ou 6 chaînons ayant un ou plusieurs hétéroatomes qui peuvent être identiques ou différents et sont choisis dans le groupe constitué par un atome d'oxygène, un atome de soufre et un atome d'azote, ledit noyau hétérocyclique substitué ayant un ou plusieurs substituants qui peuvent être identiques ou différents et sont choisis dans le groupe constitué par les atomes d'halogène, un groupe nitro, un groupe cyano, les groupes alkyle en C₁-C₆, les groupes halogéno(alkyle en C₁-C₆), les groupes alcoxy en C₁-C₆, les groupes halogéno(alcoxy en C₁-C₆), les groupes (alkyle en C₁-C₆) thio, les groupes halogéno(alkyle en C₁-C₆) thio, les groupes alcényle en C₂-C₆ et les groupes alcynyle en C₂-C₆, et R² est un groupe représenté par la formule:
-N(R²¹)R²²
dans laquelle R²¹ est un atome d'hydrogène et R²² est choisi parmi (2) des groupes alkyle en C₁-C₁₂, (3) des groupes halogéno(alkyle en C₁-C₁₂) non-substitués, (4) des groupes halogéno (alkyle en C₁-C₁₂) substitués ayant un groupe hydroxyle ou un groupe alcoxy en C₁-C₆ comme substituant, (5) des groupes alcényle en C₂-C₁₂, (6) des groupes alcynyle en C₂-C₁₂, (7) des groupes (alcoxy en C₁-C₁₂) (alkyle en C₁-C₆), (8) des groupes (alkyle en C₁-C₁₂) thio-(alkyle en C₁-C₆), (9) des groupes cyano(alkyle en C₁-C₁₂), (10) des groupes cyano(alkyle en C₁-C₆) substitués ayant sur la chaîne alkyle un susbtituant choisi parmi le groupe constitué par les groupes alcoxy en C₁-C₆, les groupes alcényloxy en C₂-C₆, les groupes alcynyloxy en C₂-C₆, les groupes (alkyle en C₁-C₆) thio, les groupes (alcoxycarbonyle en C₁-C₆) (alcoxy en C₁-C₆) les groupes (alcoxycarbonyle en C₁-C₆) (alkyle en C₁-C₆) thio, un groupe phénoxy, un groupe phénylthio et un groupe pyrazol-1-yle, (13) des groupes hydroxy(alkyle en C₁-C₆), (14) des groupes di (alcoxy en C₁-C₆) (alkyle en C₁-C₆) dans lesquels les groupes alcoxy en C₁-C₆ peuvent être identiques ou différents, (15) des groupes (alcoxy en C₁-C₆) carbonyle-(alkyle en C₁-C₆) non-substitués, (16) des groupes (alcoxy en Ci-C₆) carbonyle-(alkyle en C₁-C₆) substitués ayant sur la chaîne alkyle un substituant choisi parmi le groupe constitué par les groupes alkyle en C₁-C₆, les groupes alcoxy en C₁-C₆, les groupes alcényloxy en C₂-C₆, les groupes alcynyloxy en C₂-C₆, les groupes (alkyle en C₁-C₆) thio, les groupes (alcoxy en C₁-C₆) carbonyle-(alcoxy en C₁-C₆) , les groupes (alcoxy en C₁-C₆) carbonyle- (alkyle en C₁-C₆) thio, un groupe phényle, un groupe phényl(alkyle en C₁-C₆), un groupe phénoxy, un groupe phénylthio et un groupe pyrazol-1-yle, (17) des groupes phényle non-substitués, (18) des groupes phényle substitués ayant un ou plusieurs substituants qui peuvent être identiques ou différents et sont choisis parmi le groupe constitué par 1) les atomes d'halogène, 2) un groupe nitro, 3) un groupe cyano, 4) les groupes alkyle en C₁-C₆, 5) les groupes halogéno (alkyle en C₁-C₆), 6) les groupes alcoxy en C₁-C₆, 7) les groupes halogéno (alcoxy en C₁-C₆), 8) les groupes (alkyle en C₁-C₆) thio, 9) les groupes halogéno (alkyle en C₁-C₆) thio, 10) les groupes alcényle en C₂-C₆, 11) les groupes alcynyle en C₂-C₆, 12) les groupes (alkyle en C₁-C₆) carbonyle, 13) un groupe carboxyle, 14) les groupes (alcoxy en C₁-C₁₂) carbonyle, 15) un groupe méthylènedioxy, 16) un groupe phényle, 17) les groupes représentés par la formule : 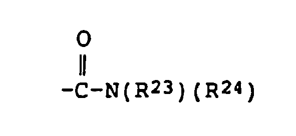 dans laquelle R²³ et R²⁴, qui peuvent être identiques ou différents, sont des atomes d'hydrogène ; des groupes alkyle en C₁-C₁₂; des groupes halogéno (alkyle en C₁-C₁₂); des groupes alcényle en C₂-C₁₂; des groupes alcynyle en C₂-C₁₂; des groupes phényle non-substitués ; des groupes phényle susbtitués ayant un ou plusieurs substituants qui peuvent être identiques ou différents et sont choisis parmi le groupe constitué par les atomes d'halogène, un groupe cyano, un groupe nitro, les groupes alkyle en C₁-C₆ et les groupes alcoxy en C₁-C₆; ou des noyaux hétérocycliques à 5 ou 6 chaînons contenant, entre des atomes de carbone adjacents de la chaîne carbonée, un ou plusieurs atomes qui peuvent être identiques ou différents et sont choisis parmi le groupe constitué par un atome d'oxygène, un atome de soufre et un atome d'azote, 18) des groupes représentés par la formule:
-SO₂-R²⁵
dans laquelle R²⁵ est un atome d'hydrogène, un groupe hydroxyle, un groupe alkyle en C₁-C₁₂, un groupe halogéno(alkyle en C₁-C₆), un groupe phényle non-substitué, un groupe phényle substitué ayant comme substituant(s) un ou plusieurs substituants qui peuvent être identiques ou différents et sont choisis parmi le groupe constitué par les atomes d'halogène, les groupes alkyles en C₁-C₆ et les groupes alcoxy en C₁-C₆, un groupe 1,2,3-thiadiazol-5-yle non-substitué, ou les groupes 1,2,3-thiadiazol-5-yle substitués ayant un atome d'halogène, un groupe alkyle en C₁-C₆ ou un groupe alcoxy en C₁-C₆ comme substituant ; 19) des groupes représentés par la formule : 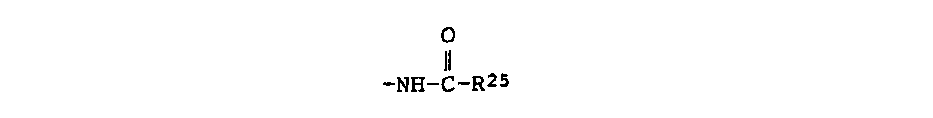 dans laquelle R²⁵ est tel que défini ci-dessus, 20) des groupes représentés par la formule: 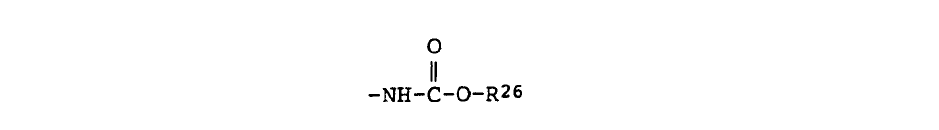 dans laquelle R²⁶ est un groupe alkyle en C₁-C₆, un groupe phényle ou un groupe phényl(alkyle en C₁-C₆), 21) des groupes représentés par la formule:
-NH-SO₂-R²⁵
dans laquelle R²⁵ est tel que défini ci-dessus, 22) des groupes représentés par la formule: 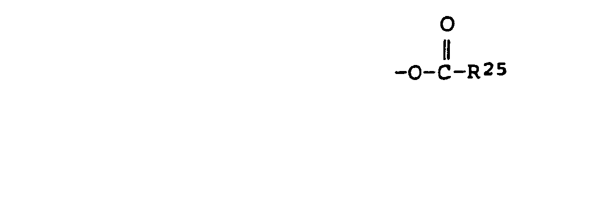 dans laquelle R²⁵ est tel que défini ci-dessus, 23) des groupes représentés par la formule : 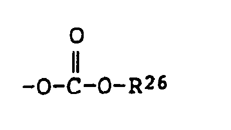 dans laquelle R²⁶ est tel que défini ci-dessus, (19) des groupes phényl(alkyle en C₁-C₆) non-substitués, (20) des groupes phényl(alkyle en C₁-C₆) substitués ayant sur le noyau un ou plusieurs substituants qui peuvent être identiques ou différents et sont choisis parmi le groupe constitué par les atomes d'halogène, un groupe nitro, un groupe cyano, les groupes alkyle en C₁-C₆, les groupes halogéno(alkyle en C₁-C₆), les groupes alcoxy en C₁-C₆, les groupes halogéno (alcoxy en C₁-C₆), les groupes (alkyle en C₁-C₆) thio, les groupes (halogénoalkyle en C₁-C₆) thio, les groupes alcényle en C₂-C₆ et les groupes alcynyle en C₂-C₆, (21) des groupes naphtyle, (22) des groupes représentés par la formule :
- (CH₂)ₚNHR¹⁸
dans laquelle R¹⁸ est tel que défini ci-dessus, et p est un nombre entier de 1 à 12, ou (23) des noyaux hétérocycliques à 5 ou 6 chaînons ayant un ou plusieurs hétéroatomes qui peuvent être identiques ou différents et sont choisis parmi le groupe constitué par un atome d'oxygène, un atome de soufre et un atome d'azote, R²¹ et R²² pouvant être pris ensemble pour représenter (24) un groupe alkylène en C₄-C₆ qui peut contenir un atome d'oxygène entre des atomes de carbone adjacents de la chaîne carbonée, ou (25) un groupe diaminométhylène pour lutter contre les maladies dans les domaines de l'agriculture et de l'horticulture qui abîment les cultures, les arbres fruitiers, les légumes, les fleurs, et les plantes ornementales.

2. Utilisation d'un agent de lutte contre les maladies dans les domaines agricole et horticole selon la revendication 1, dans laquelle R¹ représente (1) un atome d'hydrogène, (2) un groupe alkyle en C₁-C₁₂, (3) un groupe halogéno (alkyle en C₁-C₁₂), (4) un groupe alcényle en C₂-C₁₂, (5) un groupe halogéno(alcényle en C₂-C₁₂), (6) un groupe alcynyle en C₂-C₁₂, (7) un groupe halogéno (alcynyle en C₂-C₁₂), (8) un groupe cycloalkyle en C₃-C₆, (9) un groupe phényle non substitué, ou (10) un groupe phényle substitué ayant un ou plusieurs substituants qui peuvent être identiques ou différents et sont choisis dans le groupe constitué par les atomes d'halogène, un groupe nitro, un groupe cyano, les groupes alkyle en C₁-C₆, les groupes halogéno(alkyle en C₁-C₆), les groupes alcoxy en C₁-C₆, les groupes halogéno (alcoxy en C₁-C₆), les groupes (alkyle en C₁-C₆) thio, les groupes halogéno (alkyle en C₁-C₆) thio, les groupes alcényle en C₂-C₆, et les groupes alcynyle en C₂-C₆, et R² est un groupe représenté par la formule :
-N(R²¹)R²²
dans laquelle :
R²¹ est un atome d'hydrogène et R²² est choisi parmi (2) les groupes alkyle en C₁-C₁₂, (3) les groupes halogéno(alkyle en C₁-C₁₂) non-substitués, (5) les groupes alcényle en C₂-C₁₂, (6) les groupes alcynyle en C₂-C₁₂, (7) les groupes (alcoxy en C₁-C₁₂) (alkyle en C₁-C₆), (8) les groupes (alkyle en C₁-C₁₂) thio-(alkyle en C₁-C₆), (9) les groupes cyano(alkyle en C₁-C₁₂), (13) les groupes hydroxy (alkyle en C₁-C₆), (15) les groupes (alcoxy en C₁-C₆) carbonyle-(alkyle en C₁-C₆) non-substitués, (17) les groupes phényle non-substitués, (18) les groupes phényle substitués ayant un ou plusieurs substituants qui peuvent être identiques ou différents et sont choisis parmi le groupe constitué par 1) les atomes d'halogène, 2) un groupe nitro, 3) un groupe cyano, 4) les groupes alkyle en C₁-C₆, 5) les groupes halogéno(alkyle en C₁-C₆), 6) les groupes alcoxy en C₁-C₆, 7) les groupes halogéno(alcoxy en C₁-C₆), 8) les groupes (alkyle en C₁-C₆) thio, 12) les groupes (alkyle en C₁-C₆) carbonyle, 13) un groupe carboxyle, 14) les groupes (alcoxy) en C₁-C₁₂) carbonyle, 15) un groupe méthylènedioxy, 16) un groupe phényle, 17) les groupes représentés par la formule : 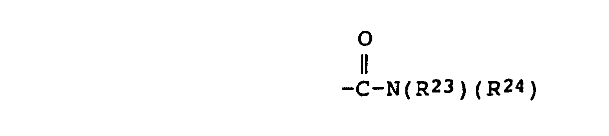
dans laquelle R²³ et R²⁴, qui peuvent être identiques ou différents, sont des atomes d'hydrogène ; des groupes alkyle en C₁-C₁₂ ; des groupes halogéno (alkyle en C₁-C₁₂) ; des groupes alcényle en C₂-C₁₂ ; des groupes alcynyle en C₂-C₁₂; des groupes phényle non-substitués ; des groupes phényle susbtitués ayant un ou plusieurs substituants qui peuvent être identiques ou différents et sont choisis parmi le groupe constitué par les atomes d'halogène, un groupe cyano, un groupe nitro, les groupes alkyle en C₁-C₆ et les groupes alcoxy en C₁-C₆; ou des noyaux hétérocycliques à 5 ou 6 chaînons contenant, entre des atomes de carbone adjacents de la chaîne carbonée, un ou plusieurs atomes qui peuvent être identiques ou différents et sont choisis parmi le groupe constitué par un atome d'oxygène, un atome de soufre et un atome d'azote, 18) des groupes représentés par la formule :
-SO₂-R²⁵
dans laquelle R²⁵ est un atome d'hydrogène, un groupe hydroxyle, un groupe alkyle en C₁-C₆, un groupe halogéno (alkyle en C₁-C₆), un groupe phényle non-substitué, un groupe phényle substitué ayant comme substituant(s) un ou plusieurs substituants qui peuvent être identiques ou différents et sont choisis parmi le groupe constitué par les atomes d'halogène, les groupes alkyles en C₁-C₆ et les groupes alcoxy en C₁-C₆, un groupe 1,2,3-thiadiazol-5-yle non-substitué, ou des groupes 1,2,3-thiadiazol-5-yle substitués ayant un atome d'halogène, un groupe alkyle en C₁-C₆ ou un groupe alcoxy en C₁-C₆ comme substituant; 19) les groupes représentés par la formule: 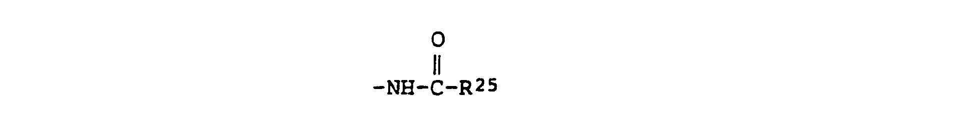 dans laquelle R²⁵ est tel que défini ci-dessus, 20) les groupes représentés par la formule : 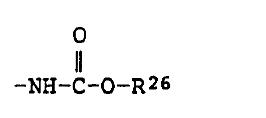 dans laquelle R²⁶ est un groupe alkyle en C₁-C₆, un groupe phényle ou un groupe phényl(alkyle en C₁-C₆), 21) les groupes représentés par la formule :
-NH-SO₂-R²⁵
dans laquelle R²⁵ est tel que défini ci-dessus, 22) les groupes représentés par la formule : 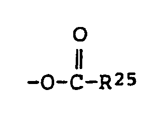 dans laquelle R²⁵ est tel que défini ci-dessus, 23) les groupes représentés par la formule: 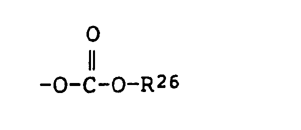 dans laquelle R²⁶ est tel que défini ci-dessus, (19) les groupes phényl(alkyle en C₁-C₆) non-substitués, (20) les groupes phényl (alkyle en C₁-C₆) substitués ayant sur le noyau un ou plusieurs substituants qui peuvent être identiques ou différents et sont choisis parmi le groupe constitué par les atomes d'halogène, un groupe nitro, un groupe cyano, les groupes alkyle en C₁-C₆, les groupes halogéno(alkyle en C₁-C₆), les groupes alcoxy en C₁-C₆, les groupes halogéno (alcoxy en C₁-C₆), (21) les groupes naphtyle, (22) les groupes représentés par la formule :
-(CH₂)ₚNHR¹⁸
dans laquelle R¹⁸ est tel que défini ci-dessus, et p est un nombre entier de 1 à 12, ou (23) les noyaux hétérocycliques à 5 ou 6 chaînons ayant un ou plusieurs atomes qui peuvent être identiques ou différents et sont choisis parmi le groupe constitué par un atome d'oxygène, un atome de soufre et un atome d'azote, R²¹ et R²² pouvant être pris ensemble pour représenter (24) un groupe alkylène en C₄-C₆ qui peut contenir un atome d'oxygène entre des atomes de carbone adjacents de la chaîne carbonée, ou (25) un groupe diaminométhylène pour lutter contre les maladies dans les domaines de l'agriculture et de l'horticulture qui abîment les cultures, les arbres fruitiers, les légumes, les fleurs, et les plantes ornementales.

3. Procédé de lutte contre les maladies des plantes qui comprend l'application d'un agent de lutte contre les maladies dans les domaines agricole et horticole présenté dans l'une quelconque des revendications 1 et 2 dans un dosage de 0, 1 g à 10 kg en termes d'ingrédient actif de l'agent de lutte contre les maladies par 10 ares afin de protéger les récoltes utiles contre la maladie.
